(19) Europäisches Patentamt
European Patent Office
Office européen des brevets

(11) EP 3 097 199 B1

(12) FASCICULE DE BREVET EUROPEEN

(45) Date de publication et mention de la délivrance du brevet:
**25.08.2021 Bulletin 2021/34**

(21) Numéro de dépôt: **15700770.9**

(22) Date de dépôt: **23.01.2015**

(51) Int Cl.:
***C12P 19/18*** (2006.01) ***C08B 37/00*** (2006.01)
***C12P 19/26*** (2006.01)

(86) Numéro de dépôt international:
**PCT/EP2015/051339**

(87) Numéro de publication internationale:
**WO 2015/110571 (30.07.2015 Gazette 2015/30)**

(54) **PROCEDE DE PRODUCTION IN VIVO DE GLYCOSAMINOGLYCANE**

VERFAHREN ZUR IN-VIVO-HERSTELLUNG VON GLYKOSAMINOGLYKANEN

METHOD FOR IN VIVO PRODUCTION OF GLYCOSAMINOGLYCANS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.01.2014 FR 1450564**

(43) Date de publication de la demande:
**30.11.2016 Bulletin 2016/48**

(73) Titulaires:
- **Centre National de la Recherche Scientifique (C.N.R.S.)**
**75016 Paris (FR)**
- **Université Grenoble Alpes**
**38400 Saint-Martin-d'Hères (FR)**

(72) Inventeur: **PRIEM, Bernard**
**F-38610 Gieres (FR)**

(74) Mandataire: **Regimbeau**
**20, rue de Chazelles**
**75847 Paris Cedex 17 (FR)**

(56) Documents cités:
**WO-A1-2010/136435 WO-A2-2008/035372**

- **JULIE A ROBINSON ET AL: "Initiation of chondroitin sulphate synthesis by beta-D-galactosides - Substrates for galactosyltransferase II", THE BIOCHEMICAL JOURNAL, vol. 227, no. 3, 1 mai 1985 (1985-05-01), pages 805-814, XP055144463,**
- **DATABASE MEDLINE [Online] US NATIONAL LIBRARY OF MEDICINE (NLM), BETHESDA, MD, US; septembre 1990 (1990-09), BRADBEER M ET AL: "The mechanism of initiation of chondroitin sulphate synthesis by beta-D-galactosides.", XP002730666, Database accession no. NLM2127885 & BIOCHEMISTRY INTERNATIONAL SEP 1990, vol. 21, no. 6, septembre 1990 (1990-09), pages 1161-1168, ISSN: 0158-5231**
- **MIKAMI TADAHISA ET AL: "Biosynthesis and function of chondroitin sulfate", BIOCHIMICA ET BIOPHYSICA ACTA (BBA) - GENERAL SUBJECTS, vol. 1830, no. 10, 1 octobre 2013 (2013-10-01), pages 4719-4733, XP028689397, ISSN: 0304-4165, DOI: 10.1016/J.BBAGEN.2013.06.006**
- **E. YAVUZ ET AL: "Glucuronylation in Escherichia coli for the bacterial synthesis of the carbohydrate moiety of nonsulfated HNK-1", GLYCOBIOLOGY, vol. 18, no. 2, 12 décembre 2007 (2007-12-12), pages 152-157, XP055144181, ISSN: 0959-6658, DOI: 10.1093/glycob/cwm134**
- **TOSHIO NINOMIYA ET AL: "Molecular cloning and characterization of chondroitin polymerase from Escherichia coli strain K4", THE JOURNAL OF BIOLOGICAL CHEMISTRY, vol. 277, no. 24, 14 juin 2002 (2002-06-14), pages 21567-21575, XP002618651, DOI: 10.1074/JBC.M201719200 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- CHUNYU ZHANG ET AL: "Metabolic engineering of Escherichia coli BL21 for biosynthesis of heparosan, a bioengineered heparin precursor", METABOLIC ENGINEERING, vol. 14, no. 5, 1 septembre 2012 (2012-09-01), pages 521-527, XP055144554, ISSN: 1096-7176, DOI: 10.1016/j.ymben.2012.06.005
- DEANGELIS P L: "Microbial glycosaminoglycan glycosyltransferases", GLYCOBIOLOGY, OXFORD UNIVERSITY PRESS, US, vol. 12, no. 1, 1 janvier 2002 (2002-01-01), pages 9R-16R, XP002333248, ISSN: 0959-6658, DOI: 10.1093/GLYCOB/12.1.9R
- PAUL L DEANGELIS: "Glycosaminoglycan polysaccharide biosynthesis and production: today and tomorrow", APPLIED MICROBIOLOGY AND BIOTECHNOLOGY, SPRINGER, BERLIN, DE, vol. 94, no. 2, 6 mars 2012 (2012-03-06), pages 295-305, XP035033397, ISSN: 1432-0614, DOI: 10.1007/S00253-011-3801-6
- None

**Description**

La présente invention a pour objet un procédé de production de glycosaminoglycane (GAG) in vivo, par ingénierie métabolique dans une cellule de bactérie génétiquement modifiée. Dans un procédé selon l'invention, ladite cellule est génétiquement modifiée dans le but d'exprimer les gènes codant pour les enzymes adéquates pour la synthèse de GAG à partir d'un précurseur exogène à la cellule, et de préférence un précurseur exogène internalisé par ladite cellule. Selon un aspect particulier, la présente invention a pour objet un procédé de production de chondroïtine ou d'héparosane dans une cellule bactérienne génétiquement modifiée, à partir d'un précurseur béta-galactoside exogène, de préférence internalisé par la cellule. Selon un autre aspect particulier, la présente invention a pour objet l'utilisation d'une cellule de *Escherichia coli* comprenant au moins les gènes *glcA-T, kfoC, kfiD* et *wbpP* pour la production de chondroïtine. Selon un autre aspect particulier, la présente invention a pour objet l'utilisation d'une cellule de *Escherichia coli* comprenant au moins les gènes *glcA-T, kfiA, kfiB kfiC et kfid* pour la production d'héparosane.

Les glycosaminoglycanes (GAGs) sont des hétéro-polysaccharides qui composent la matrice extracellulaire. Ils sont composés d'une répétition d'un motif di-saccharidique composé, d'une part, d'une hexosamine qui peut être la N-acétylglucosamine ou la N-acétylgalactosamine, et, d'autre part, d'un acide uronique, qui peut être l'acide glucuronique ou l'acide iduronique, ou d'un galactose. Ces motifs peuvent être sulfatés. Les GAGs sont fortement chargés négativement du fait de la présence des groupes sulfate ou carboxyl sur la plupart de leurs oses, ce qui leur confère des propriétés viscosantes, ils jouent un rôle dans le maintien des tissus conjonctifs et sont également impliqués dans la reconnaissance cellulaire et la croissance des tissus. Selon la composition du motif di-saccharidique, on distingue parmi les GAGs : l'acide hyaluronique, la chondroïtine, la chondroïtine sulfate, le dermatane sulfate, l'héparane sulfate, l'héparine, l'héparosane et le kératane sulfate. La chondroïtine est un précurseur des chondroïtines sulfate et des dermatanes sulfate (Cimini *et al.,* 2010). *In vivo,* la chondroïtine est sujette durant sa polymérisation à de nombreuses sulfatations, opérées par des chondroïtine sulfotransférases, qui résultent en la formation de groupes O-sulfatés en des positions variées. Selon la position de ces groupements sulfates, les chondroïtines sulfate jouent différents rôles.

Les GAGs peuvent être utilisés dans différents domaines thérapeutiques. Leur application la plus connue est le traitement des ostéoarthrites, ils possèdent une activité anti-inflammatoire et une action dans la régénération des cartilages. Plus récemment, il a été démontré que les chondroïtines sulfate ont des effets anti-viraux et anti-infectieux, elles sont également utilisées dans la régénération et l'ingénierie de tissus. Les chondroïtines sulfate sont aussi étudiées pour leur intérêt dans le diagnostic et le traitement de cancer (Jin *et al.,* 2008 ; Yamada et Sugahara, 2011). De plus, la chondroïtine peut être utilisée comme adjuvant nutritionnel. L'héparosane, dont le squelette est un précurseur de la synthèse de l'héparine, peut être utilisé dans différentes applications thérapeutiques, en tant que précurseur d'héparine non animale ou de produits analogues de l'héparine, mais aussi pour la vectorisation de produits thérapeutiques.

Les GAGs peuvent être extraits à partir de sources animales ou synthétisés par voie chimique, enzymatique ou microbiologique. La synthèse de ces composés par chimie organique traditionnelle reste cependant à ce jour très difficile. La chondroïtine sulfate et l'acide hyaluronique peuvent être extraits et purifiés à partir de tissu animal tel que les ailerons de requin, le cartilage de poisson ou les carcasses d'animaux. L'utilisation, notamment thérapeutique, de ces composés requiert des niveaux de qualité et de purification élevés et constants que l'extraction ne peut satisfaire pleinement, notamment du fait du risque de contamination des GAGs extraits de tissus animaux, par exemple par un virus ou un prion (Zanfardino *et al.,* 2010 ; Wang *et al.,* 2011) .

Une autre source de GAGs est leur extraction de certaines bactéries pathogènes, où ils font partie de la capsule extracellulaire, ce qui leur confère un rôle de barrière naturelle face aux défenses de l'hôte. La production de chondroïtine à partir de polysaccharides capsulaires (Capsular Poly-Saccharides) a déjà été réalisée à partir des souches de *E. coli* pathogènes K4 et K5 (Schiraldi *et al.,* 2010 ; Inoué *et al.,* 1990). Cependant, la plupart des utilisations thérapeutiques de la chondroïtine nécessitent une taille de 14 à 20 kDa (Schiraldi *et al.,* 2010). Les tailles moyennes des CPS sauvages sont d'environ 650 kDa et celles des GAGs produits par ingénierie métabolique sont d'environ 150 kDa (Krahulec *et al.,* 2005), la chondroïtine ainsi obtenue doit donc être dépolymérisée préalablement à la plupart de ses utilisations thérapeutiques. Cette étape de dépolymérisation diminue l'homogénéité du produit et sa qualité d'un point de vue thérapeutique.

Il existe donc actuellement un besoin de disposer d'un procédé de production de glycosaminoglycanes en qualité et en quantité appropriée pour un usage notamment thérapeutique. Ceci est maintenant l'objet de la présente invention.

Les inventeurs ont en effet mis au point un procédé d'ingénierie par modification génétique d'une cellule permettant de produire des glycosaminoglycanes, et notamment de la chondroïtine et de l'héparosane, à partir d'un accepteur exogène de type béta-galactoside. Cet accepteur exogène à deux unités saccharidiques remplace l'accepteur naturel bactérien, ou accepteur « endogène ». Selon un mode de réalisation de l'invention, ledit « accepteur exogène » peut être internalisé par la cellule. Selon un autre mode de réalisation de l'invention, ledit « accepteur exogène » peut être l'objet d'une synthèse intracellulaire par une bétagalactosyltransférase recombinante.

L'état de l'art ne décrit pas le béta-galactoside comme accepteur possible des enzymes possédant une activité de synthèse des GAGs. La biosynthèse des glycosaminoglycanes bactériens prend sa source dans le cytoplasme des bactéries, au voisinage de la membrane par laquelle ils transitent afin d'intégrer la paroi extracellulaire, à partir d'un accepteur membranaire endogène, vraisemblablement le diacylglycérophosphate (Whitfield, 2006). Il a été montré dans un système d'expression bactérien *in vitro* qu'en ajoutant dans le milieu réactionnel des oligosaccharides dont la taille minimale est de 6 unités saccharidiques, et en présence des nucléotide-sucres UDP-GlcA et UDP-GalNAc, la chondroïtine polymérase KfoC d'Escherichia coli K-4 qui possède les deux activités GlcA-transférase et GalNAc- transférase est capable de produire un polysaccharide d'une taille d'environ 20 kDa (Ninomiya *et al,* 2002). WO 01/04341 et Priem *et al.* (2002) décrivent la production d'oligosaccharides dans une souche bactérienne capable d'intégrer un précurseur exogène. Yavuz *et al.* (2008) décrit la production d'oligosaccharides glucuronylés dans E. Coli. Doherty et al. (US2011/0244520) ont décrit un procédé de production de chondroïtine dans une souche bactérienne utilisant un ensemble de gènes exogènes, mais en absence d'un précurseur exogène. Robinson et al. (The Biochemical Journal, vol. 227, no. 3, pages 805-814, 1er mai 1985) divulgue un procédé de production de chondroïtine sulfate dans une culture in vitro de cellules de cartilage d'embryon de poussin contenant des chondrocytes, en présence de béta-galactoside exogène.

Les inventeurs ont maintenant démontré que, sous réserve de la présence dans la bactérie d'un glucuronyl béta-galactoside et d'au moins une enzyme comportant une activité de synthèse d'un GAG à partir de glycosyl-béta galactoside, il est possible de réaliser la synthèse par ingénierie métabolique de GAG de polydispersité réduite et de taille adaptée à un usage thérapeutique (Figures 1 et 2). En particulier, les inventeurs ont démontré la possibilité de produire de la chondroïtine, par ingénierie métabolique, sous réserve de la présence dans la bactérie d'un glucuronyl-béta galactoside et d'une chondroïtine synthase bactérienne, et en présence d'un accepteur exogène béta-galactoside. Les inventeurs ont également démontré qu'en présence d'un accepteur exogène béta-galactoside et d'au moins un gène codant pour une enzyme capable de synthétiser l'héparosane, et de préférence les gènes *kfiA, kfiB, kfiC* et *kfiD,* il est possible de produire de l'héparosane, avec un rendement supérieur aux rendements obtenus par la mise en œuvre des procédés déjà connus.

La synthèse de GAG à partir d'un accepteur exogène par un procédé selon l'invention a pour avantage de permettre de transposer la voie de biosynthèse dans différents types de cellules capable d'internaliser ledit précurseur exogène. De ce fait, la présence de l'accepteur endogène membranaire propre à *E. coli* ou aux souches bactériennes pathogènes productrices de GAGs n'est pas nécessaire. Le GAG ainsi produit se caractérise par la présence en fin de chaine du précurseur exogène béta-galactoside, ce motif s'apparente à un motif naturellement présent chez l'homme (Kakuda *et al.,* 2004).

Un procédé de production de GAG par voie métabolique selon l'invention présente également un intérêt économique en raison du faible coût du précurseur exogène utilisé.

L'invention sera maintenant décrite de manière détaillée et illustrée d'exemples.

La présente invention a pour objet un procédé de production de glycosaminoglycane (GAG) dans une cellule génétiquement modifiée, ledit procédé comprenant les étapes suivantes:

1) l'obtention d'une cellule comprenant au moins :

- un gène exogène codant pour une enzyme de mammifère capable d'exercer une activité glucuronyl transférase sur un substrat béta-galactoside, et les éléments permettant l'expression dudit gène dans ladite cellule et la synthèse de glucuronyl béta-glucuronide,
- un gène codant pour une enzyme capable de synthétiser ledit glycosaminoglycane à partir du glucuronyl béta-galactoside, ou les gènes codant pour les enzymes de la voie de synthèse dudit glycosaminoglycane à partir du glucuronyl béta-galactoside, et les éléments permettant l'expression dudit ou desdits gènes dans ladite cellule,
- un gène codant pour une enzyme capable d'assurer l'internalisation de béta-galactoside exogène par la cellule, et les éléments permettant l'expression dudit gène dans ladite cellule, et

2) la culture de ladite cellule en présence de béta-galactoside exogène et dans des conditions compatibles avec la production dudit GAG par la cellule, dans lequel ladite cellule est une cellule de bactérie.

Le terme « glycosaminoglycane » (GAG) désigne un hétéro-polysaccharide composé d'une répétition d'un motif di-saccharidique composé d'une hexosamine et d'un acide uronique ou d'un galactose. Selon un aspect particulier, un procédé selon l'invention a pour objet la production dans une cellule génétiquement modifiée d'un GAG choisi dans le groupe constitué par : l'acide hyaluronique, la chondroïtine, la chondroïtine sulfate, le dermatane, le dermatane sulfate, l'héparane, l'héparane sulfate, l'héparine, l'héparosane, le kératane et le kératane sulfate.

Le terme « cellule génétiquement modifiée » désigne un micro-organisme dont la séquence du génome a subi au moins une altération, une addition ou une délétion, afin de conférer un phénotype particulier à ladite cellule. Cette modification peut notamment être apportée par l'addition d'au moins un gène exogène et des éléments permettant l'expression de ce gène dans ladite cellule. Selon un aspect particulier d'un procédé selon l'invention, ladite cellule est génétiquement modifiée par l'introduction d'au moins un gène exogène porté par un plasmide. Selon un autre aspect particulier d'un procédé selon l'invention, ladite cellule est génétiquement modifiée par l'introduction d'au moins un gène exogène intégré dans le génome de ladite cellule.

Le terme « béta-galactoside exogène » désigne un composé précurseur, internalisé par ladite cellule ou objet d'une synthèse intracellulaire par une béta-galactosyltranférase recombinante, intervenant dans la voie de biosynthèse du GAG et substrat d'une enzyme ayant une activité glycosyl-transférase, de préférence une activité glucuronyl-transférase.

Le terme « gène exogène » désigne un acide nucléique hétérologue, non naturellement présent dans la cellule, dont la séquence en nucléotides code pour une protéine définie. Ledit acide nucléique peut être de l'ADN, sous une forme simple brin ou double brin, ou de l'ARN. Dans un procédé selon l'invention, ledit gène exogène est introduit dans ladite cellule par les techniques bien connues de l'homme du métier spécialiste de la manipulation des acides nucléiques recombinants, telles que, par exemple, le choc thermique ou l'électroporation.

Le terme « éléments permettant l'expression dudit gène dans ladite cellule » désigne notamment un promoteur, des signaux d'initiation et de terminaison de la traduction, ainsi que des régions appropriées de régulation de la transcription. Selon un mode de réalisation préféré de l'invention, la séquence d'acide nucléique est introduite dans ladite cellule sous la forme d'un vecteur d'expression qui comporte lesdits éléments permettant l'expression dudit gène dans ladite cellule. Un vecteur d'expression peut également comprendre un ou des éléments permettant son maintien dans la cellule, de façon stable au cours des générations successives. Ces éléments sont choisis en fonction de la cellule hôte utilisée, et peuvent être, notamment un gène codant pour la résistance à un antibiotique. Ledit vecteur peut posséder une réplication autonome ou bien s'intégrer dans le génome de la cellule hôte. Selon un mode particulier de réalisation de l'invention, le vecteur d'expression est présent sous forme de plasmide, un plasmide pouvant porter un ou plusieurs des gènes exogènes d'intérêt pour la mise en œuvre d'un procédé selon l'invention. Chacun des plasmides présents dans la cellule peut être caractérisé par le nombre de copies dudit plasmide dans la cellule. Selon un aspect particulier d'un procédé selon l'invention, un plasmide portant au moins un gène exogène d'intérêt pour la mise en œuvre d'un procédé selon l'invention est présent à un faible nombre de copies (« low copy »). Selon un autre aspect particulier d'un procédé selon l'invention, un plasmide portant au moins un gène exogène d'intérêt pour la mise en œuvre d'un procédé selon l'invention est présent à un fort nombre de copies (« high copy »). Il est bien connu d'un homme du métier spécialiste du domaine technique que le niveau d'expression d'un gène peut être corrélé au nombre de copies du gène dans la cellule.

L'expression « enzymes de la voie de synthèse dudit glycosaminoglycane à partir du glucuronyl béta-galactoside » désigne les différentes enzymes capables d'exercer une activité intervenant au cours de la voie de synthèse du GAG dans la cellule.

Selon un aspect particulier d'un procédé selon l'invention, ledit béta-galactoside exogène est présent dans le milieu de culture. Selon un autre aspect particulier d'un procédé selon l'invention, ledit béta-galactoside exogène est du béta-galactoside recombinant, produit par ladite cellule génétiquement modifiée, ladite cellule génétiquement modifiée comprenant au moins un gène exogène permettant la synthèse de béta-galactoside. Selon un mode de réalisation préféré d'un procédé selon l'invention, le béta-galactoside est choisi dans le groupe constitué par les béta-galactosides pris en charge par le système de transport actif de la bactérie, et de préférence dans le groupe constitué par : le lactose, le lactose-allyle, le béta-allyle-galactoside, le béta-méthylgalactoside, le béta-galactoside d'allyle, le béta-galactoside de propargyle.

Selon un aspect particulier d'un procédé selon l'invention, la culture de ladite cellule génétiquement modifiée est réalisée en présence de béta-galactoside exogène, ledit béta-galactoside exogène étant ajouté durant la culture de façon à ce que le glucuronyl béta-galactoside, obtenu par le transfert d'un groupe glucuronyl sur le béta-galactoside, soit présent dans le compartiment intracellulaire de la cellule pendant la plus grande partie de la culture, et de préférence pendant toute la durée de la culture. Le béta-galactoside peut être ajouté ponctuellement dans la culture ou sous forme d'ajout en goutte à goutte. Le béta-galactoside peut être ajouté à la culture à une concentration comprise entre environ 0,5 et environ 8 g/L, le terme « environ » exprimant une variation de l'ordre de 10%, de préférence entre environ 0,6 et environ 7 g/L, de préférence entre environ 1 et environ 5 g/L, et plus préférentiellement entre environ 1,5 et environ 4 g/L.

L'invention a également pour objet un procédé de production de GAG dans une cellule génétiquement modifiée selon l'invention dans laquelle l'enzyme capable d'assurer l'internalisation par la cellule de béta-galactoside exogène est une perméase, de préférence une perméase spécifique du lactose, de préférence la béta-galactoside perméase codée par le gène LacY de *E. coli.*

Dans un procédé de production de GAG dans une cellule génétiquement modifiée selon l'invention, la culture de ladite cellule est réalisée selon un procédé bien connu par un homme du métier, qui peut mettre en œuvre le procédé de culture et choisir la composition du milieu de culture, notamment la concentration en éléments nutritifs, et les paramètres de la culture, tels que la température, le pH, la concentration en oxygène, l'inoculation de la culture, selon ses connaissances générales. Le milieu de culture peut notamment comprendre au moins une source d'un ou plusieurs éléments choisis parmi : sucres, carbone, azote, phosphate, magnésium, calcium et vitamines. Dans un procédé selon l'invention, la culture peut être réalisée en fermenteur selon un procédé de fermentation conventionnel choisi parmi : la culture en batch, la culture en fed-batch ou la culture continue. De préférence, dans un procédé selon l'invention, la culture de ladite cellule génétiquement modifiée est réalisée en fed-batch.

Selon l'invention, la glucuronyl transférase est une glucuronyl transférase de mammifère. De préférence, la glucuronyl-transférase de mammifère est choisie est une glucuronyl transférase de rat ou une glucuronyl transférase de souris, et de préférence la glucuronyl-transférase de cerveau de souris.

Selon un aspect encore plus particulier, l'invention a pour objet un procédé de procédé de production de glycosaminoglycanes (GAGs) dans une cellule génétiquement modifiée selon l'invention, dans lequel le gène codant pour une enzyme capable d'exercer une activité glucuronyl-transférase sur un substrat béta-galactoside est le gène *glcA-T* (codant pour la béta-1-3-glucuronyl transférase de souris (Référence GenBank GI : 568960207 du 27 décembre 2013).

Selon un mode de réalisation particulier, l'invention a pour objet un procédé de production de GAG dans une cellule génétiquement modifiée, caractérisée en ce que le GAG est produit dans le cytoplasme de ladite cellule.

L'invention a également pour objet un procédé de production de GAG dans une cellule génétiquement modifiée selon l'invention, dans laquelle ladite cellule comprend en outre un gène codant pour une enzyme capable de synthétiser l'UDP-GlcA à partir d'UDP-Glc, et les éléments permettant l'expression dudit gène dans ladite cellule. Selon un mode de réalisation particulier de l'invention, l'enzyme capable de synthétiser l'UDP-GlcA à partir d'UDP-Glc est l'UDP-glucose déshydrogénase.

L'invention a également pour objet un procédé de production de GAG dans une cellule génétiquement modifiée selon l'invention, dans laquelle ladite cellule génétiquement modifiée comprend en outre un gène codant pour une enzyme capable de synthétiser l'UDP-GalNAc, et les éléments permettant l'expression dudit gène dans ladite cellule. Selon un mode de réalisation particulier de l'invention, l'enzyme capable de synthétiser l'UDP-GalNAc est l'UDP-N-acétyl-glucosamine épimérase de Pseudomonas aeroginosa. Selon un autre mode de réalisation particulier, l'enzyme capable de synthétiser l'UDP-GalNAc est l'UDP-GlcNAc/Glc 4-epimerase (EC:5.1.3.2) de Campylobacter jejuni subsp. jejuni NCTC 11168 = ATCC 700819, référencée sous K01784, UDP-glucose 4-epimerase (EC:5.1.3.2).

L'invention a également pour objet un procédé de production de GAG dans une cellule génétiquement modifiée selon l'invention dans laquelle au moins un gène de ladite cellule génétiquement modifiée codant pour une enzyme utilisant l'UDP-glcA pour la synthèse d'un produit cellulaire est inactivé, et dans lequel ladite cellule est une bactérie de type *Escherichia coli* et le gène *wcaJ* codant pour l'enzyme utilisant l'UDP-glcA pour la synthèse de l'acide colanique est inactivé.

L'invention a également pour objet un procédé de production de GAG dans une cellule génétiquement modifiée selon l'invention dans laquelle au moins un gène de ladite cellule génétiquement modifiée codant pour une enzyme capable d'hydrolyser le béta-galactoside est inactivé, et dans lequel ladite cellule est une bactérie de type *Escherichia coli* et que l'enzyme capable d'hydrolyser le béta-galactoside dont le gène est inactivé est la béta-galactosidase codée par le gène LacZ.

L'invention a également pour objet un procédé de production de GAG dans une cellule génétiquement modifiée selon l'invention dans laquelle ladite cellule génétiquement modifiée est choisie dans le groupe constitué par : *Escherichia coli, Bacillus subtilis, Campylobacter pylori, Helicobacter pylori, Agrobacterium tuméfaciens, Staphylococcus aureus, Thermophilus aquaticus, Azorhizobium caulinodans, Rhizobium leguminosarum, Rhizobium meliloti, Neisseria gonorrhoeae, Neisseria meningitidis.*

Selon un mode de réalisation préféré de l'invention, ladite cellule génétiquement modifiée est une bactérie de souche *Escherichia coli.* Selon un mode de réalisation encore préféré de l'invention, ladite cellule génétiquement modifiée est une bactérie de souche *Escherichia coli* non pathogène. Plus particulièrement, ladite cellule génétiquement modifiée est choisie dans le groupe constitué par : la souche *Escherichia coli* K12, la souche DH1 de *Escherichia coli* K12 (référence ATCC : 68153), la souche DH1 de Escherichia coli K12 dans laquelle les gènes lacZ et wcaJ sont inactivés (Yavuz *et al.,* 2008), une souche *Escherichia coli* du groupe B, de préférence *Escherichia coli BL21.*

Selon un mode de réalisation particulier, l'invention a pour objet un procédé de production de GAG dans une cellule génétiquement modifiée, caractérisé en ce que ledit GAG est la chondroïtine.

Selon un mode de réalisation particulier, l'invention a pour objet un procédé de production de chondroïtine dans une cellule génétiquement modifiée, ledit procédé comprenant :

1) l'obtention d'une cellule comprenant au moins un gène exogène codant pour une enzyme de mammifère capable d'exercer une activité glucuronyl transférase sur un substrat béta-galactoside, un gène codant pour une enzyme capable de synthétiser la chondroïtine à partir de glucuronyl béta-galactoside, un gène codant pour une enzyme capable d'assurer l'internalisation par la cellule de béta-galactoside exogène, et
2) la culture de la cellule en présence de béta-galactoside exogène et dans des conditions compatibles avec la production de chondroïtine par ladite cellule, dans lequel ladite cellule est une cellule de bactérie.

Le terme « chondroïtine » désigne un GAG ayant pour motif di-saccharidique une N-acétylgalactosamine (GalNAc) et un acide glucuronique (GlcA) et peut être représenté par : « 4-GlcA-β-1,3-GalNAc-β-1 ».

Selon un mode réalisation encore plus particulier, l'invention a pour objet un procédé de production de chondroïtine dans une cellule génétiquement modifiée selon l'invention, dans lequel ledit béta-galactoside exogène est le lactose.

L'invention a également pour objet un procédé de production de chondroïtine dans une cellule génétiquement modifiée dans laquelle l'enzyme capable de synthétiser la chondroïtine est une synthase bactérienne capable de synthétiser la chondroïtine à partir de glucuronyl-lactose. Selon un aspect encore plus particulier, l'invention a pour objet un procédé de production de chondroïtine dans une cellule génétiquement modifiée dans lequel le gène codant pour une enzyme capable d'exercer une activité de synthèse de la chondroïtine est le gène *kfoC* codant pour la chondroïtine polymérase de la souche K4 de *E. coli* PMV-1 (Référence GenBank : GI : 21326784 du 20 décembre 2010).

Selon un autre mode de réalisation particulier, l'invention a pour objet un procédé de production de GAG dans une cellule génétiquement modifiée, caractérisé en ce que ledit GAG est l'héparosane.

Selon un mode de réalisation particulier, l'invention a pour objet un procédé de production d'héparosane dans une cellule génétiquement modifiée, ledit procédé comprenant :

- l'obtention d'une cellule comprenant au moins un gène exogène codant pour une enzyme de mammifère capable d'exercer une activité glucuronyl transférase sur un substrat béta-galactoside, les gènes codant pour les enzymes de la voie de synthèse dudit glycosaminoglycane à partir du glucuronyl béta-galactoside, et les éléments permettant l'expression desdits gènes dans ladite cellule un gène codant pour une enzyme capable d'assurer l'internalisation par la cellule de béta-galactoside exogène, et
- la culture de ladite cellule en présence de béta-galactoside exogène, et dans des conditions compatibles avec la production d'héparosane par ladite cellule, dans lequel ladite cellule est une cellule de bactérie.

Le terme "héparosane" désigne un glycosaminoglycane bactérien composé de répétitions régulières de 4-β-glucuronyl-(1-4)-α-N-acetylglucosaminyl-1. L'héparosane est produit par des bactéries pathogènes telles que *Escherichia coli* K5 et *Pasteurella multocida* et trouvé dans la capsule bactérienne.

Selon un autre mode de réalisation encore plus particulier, l'invention a pour objet un procédé de production d'héparosane dans une cellule génétiquement modifiée selon l'invention, caractérisée en ce que ledit béta-galactoside est le lactose.

Selon un autre mode de réalisation encore plus particulier, l'invention a pour objet un procédé de production d'héparosane dans une cellule génétiquement modifiée selon l'invention, caractérisée en ce que ledit béta-galactoside est l'allyl-lactose.

Selon un aspect particulier, l'invention a pour objet un procédé de production de chondroïtine dans une cellule génétiquement modifiée selon l'invention, le procédé comprenant l'obtention d'une cellule de la souche K12 *d'Escherichia coli,* comprenant au moins :

- un gène codant pour une glucuronyl transférase de mammifère, et les éléments permettant l'expression dudit gène dans ladite cellule,
- un gène codant pour une chondroïtine synthase bactérienne, et les éléments permettant l'expression dudit gène dans ladite cellule,
- un gène codant pour une béta-galactoside perméase, et les éléments permettant l'expression dudit gène dans ladite cellule,

  et la culture de ladite cellule en présence de béta-galactoside exogène et dans des conditions compatibles avec la production de chondroïtine par la cellule.

Selon un aspect encore plus particulier de l'invention, le procédé de production de chondroïtine comprend l'obtention d'une cellule de la souche K12 *d'Escherichia coli,* comprenant au moins :

- un gène codant pour la glucuronyl transférase de souris, et les éléments permettant l'expression dudit gène dans ladite cellule,
- un gène codant pour la chondroïtine synthase *kfoC* de E. coli K4, et les éléments permettant l'expression dudit gène dans ladite cellule,
- un gène codant pour une lactose perméase, et les éléments permettant l'expression dudit gène dans ladite cellule,
- un gène codant pour une UDP-glucose deshydrogénase, et
- un gène codant pour l'UDP-N-acetyl-glucosamine épimérase de *Pseudomonas aeroginosa,*

  et la culture de ladite cellule en présence de béta-galactoside exogène et dans des conditions compatibles avec la production dudit GAG par la cellule.

Selon un aspect encore plus particulier, l'invention a pour objet un procédé de production de chondroïtine dans une cellule génétiquement modifiée, ledit procédé comprenant l'obtention d'une cellule de la souche K12 *d'Escherichia coli,* ladite cellule comprenant au moins les gènes *glcA-T* (*GI: 568960207), kfoC* (GI: 21326784), *kfid* (UDP-glucose-6-dehydrogenase, E coli, GI 735963 du 23 octobre 2008) et *wbpP* (Pseudomonas aeruginosa, GI : 6690131 du 12 janvier 2000) et la culture de ladite cellule en présence de lactose exogène et dans des conditions compatibles avec la production de chondroïtine par la cellule.

L'invention a également pour objet un procédé de production d'héparosane dans une cellule génétiquement modifiée selon l'invention, comprenant l'obtention d'une cellule comprenant au moins : un gène codant pour une enzyme de mammifère capable d'exercer une activité glucuronyl transférase sur un substrat béta-galactoside, les gènes codant pour les enzymes de la voie de synthèse dudit glycosaminoglycane à partir du glucuronyl béta-galactoside, et les éléments permettant l'expression dudit ou desdits gènes dans ladite cellule, un gène codant pour une enzyme capable d'assurer l'internalisation de béta-galactoside exogène par la cellule, et les éléments permettant l'expression dudit gène dans ladite cellule, et la culture de ladite cellule en présence de béta-galactoside exogène et dans des conditions compatibles avec la production d'héparosane par la cellule, dans lequel ladite cellule st une cellule de bactérie.

Selon un mode réalisation encore plus particulier, l'invention a pour objet un procédé de production d'héparosane dans une cellule génétiquement modifiée selon l'invention, dans lequel ledit béta-galactoside exogène est le lactose.

L'invention a plus particulièrement pour objet un procédé de production d'héparosane dans une cellule génétiquement modifiée selon l'invention, comprenant l'obtention d'une cellule comprenant au moins un gène exogène codant pour une GlcNAc transférase, un gène exogène codant pour une GlcA transférase, et un gène exogène codant pour une UDP-Glc deshydrogénase.

Selon un mode de réalisation encore plus particulier, l'invention a également pour objet un procédé de production d'héparosane dans une cellule génétiquement modifiée selon l'invention, comprenant l'obtention d'une cellule de souche Escherichia coli, et de préférence la souche K12, ladite cellule comprenant au moins :

- le gène glcA-T (GI 568960207 du 27 décembre 2013),
- le gène *kfiA* (GI : 496605 du 23 octobre 2008) qui code pour une GlcNAc transférase,
- le gène *hfiB* (SEQ ID N°1) qui code pour une protéine dont le rôle n'est pas connu à ce jour,
- le gène *kfiC* qui code pour une glcA transférase (GI : 735964 du 23 octobre 2008),
- le gène *kfiD* qui code pour une UDP-Glucose 6 deshydrogénase (GI : 735963 du 23 octobre 2008),

et la culture de ladite cellule en présence d'un béta-galactoside choisi parmi : le lactose et le lactose allyle, dans des conditions compatibles avec la production d'héparosane par la cellule.

Selon un mode de réalisation encore plus particulier, l'invention a également pour objet un procédé de production d'héparosane dans une cellule génétiquement modifiée selon l'invention, comprenant l'obtention d'une cellule de la souche Escherichia coli, et de préférence la souche K12 de Escherichia coli, ladite cellule comprenant au moins le gène codant pour une béta 1,3 glucuronyl transférase de mammifère et la séquence dont la référence est X77617.1 (23 octobre 2008) qui comprend les gènes *kfiD, kfiC, kfiB* et *kfiA* de E. coli et la culture de ladite cellule en présence d'un béta-galactoside choisi parmi : le lactose et le lactose allyle, dans des conditions compatibles avec la production d'héparosane par la cellule.

Selon un mode de réalisation encore plus particulier, l'invention a également pour objet un procédé de production d'héparosane dans une cellule génétiquement modifiée selon l'invention, comprenant l'obtention d'une cellule de la souche Escherichia coli, et de préférence la souche K12 de Escherichia coli, ladite cellule comprenant au moins a) le gène *glcA-T,* et b) les gènes *kfiA, kfiB kfiC et kfiD de E. coli* ou au moins un gène codant pour une protéine ayant une fonction homologue à celle d'une protéine codée par *kfiA, kfiB kfiC ou kfiD* et la culture de ladite cellule en présence d'un béta-galactoside choisi parmi : le lactose et le lactose allyle, dans des conditions compatibles avec la production d'héparosane par la cellule.

Par « protéine ayant une fonction homologue » on entend une protéine exerçant une fonction ou une activité de même nature. Les fonctions ou activités de deux ou plusieurs protéines peuvent être considérées comme homologues d'après le résultat de tests dans lesquels ces protéines sont placées dans des conditions réactionnelles identiques un même paramètre est évalué, par exemple l'apparition du produit donné d'une réaction enzymatique. Des protéines ayant une fonction homologue permettront l'obtention du même produit.

Dans un procédé selon l'invention, un gène codant pour une protéine ayant une fonction homologue à celle d'une protéine codée par *kfiA, kfiB kfiC* ou *kfiD* peut notamment être un gène homologue issu d'une autre souche bactérienne, telle que par exemple *Pasteurella multocida,* et notamment le gène *PmHS1* qui code pour une protéine possédant la double activité GlcAT et GlcNAcT.

La mise en œuvre d'un procédé selon l'invention permet de produire le GAG dans le compartiment cytoplasmique de ladite cellule modifiée. Les substrats des sulfotransférases, ou PAPS, sont également produits dans le cytoplasme des cellules. Du fait de la co-localisation possible du GAG et des PAPS, la sulfatation des GAGs est possible dans le cytoplasme desdites cellules génétiquement modifiées.

D'autres aspects ne faisant pas part de l'invention sont décrits ci-dessous. Il est décrit (non revendiqué) un procédé de production de chondroïtine sulfate dans une cellule génétiquement modifiée, ledit procédé comprenant une première étape de production du GAG et une deuxième étape de sulfatation du GAG.

La sulfatation du GAG peut être réalisée par la mise en œuvre de procédés bien connus de l'homme du métier. La synthèse de chondroïtine sulfate peut être réalisée par sulfatation chimique ou enzymatique (Ninomiya *et al.,* 2002). La sulfatation du GAG peut également être mise en œuvre au sein de la cellule génétiquement modifiée dans laquelle le GAG est produit, notamment par la présence de gènes, endogènes ou exogènes, codant pour une enzyme capable d'exercer une activité de GAG sulfotransférase.

Selon un aspect encore plus particulier, il est décrit (non revendiqué) un procédé de production de GAG dans une cellule génétiquement modifiée comprenant

1) l'obtention d'une cellule comprenant au moins :
un gène exogène codant pour une enzyme, de préférence de mammifère, capable d'exercer une activité glycosyl transférase sur un substrat béta-galactoside, de préférence une activité glucuronyl transférase, et les éléments permettant l'expression dudit gène dans ladite cellule ; un gène codant pour une enzyme capable de synthétiser ledit GAG à partir de glycosyl béta-glucuronide, ou les gènes codant pour les enzymes de la voie de synthèse dudit glycosaminoglycane à partir du glycosyl béta-glucuronide, et les éléments permettant l'expression dudit ou desdits gènes dans ladite cellule, un gène codant pour une enzyme capable d'assurer l'internalisation de béta-galactoside exogène par la cellule, et les éléments permettant l'expression dudit gène dans ladite cellule,
2) la culture de ladite cellule en présence de béta-galactoside exogène et dans des conditions compatibles avec la production d'héparosane par la cellule, et
3) la purification du GAG produit à l'étape 2).

La purification du glycosaminoglycane à partir de la culture de la cellule génétiquement modifiée peut être réalisée par tout moyen adapté connu de l'homme du métier. Ce moyen sera notamment adapté à la production de GAG dans le cytoplasme de la cellule génétiquement modifiée.

Selon un aspect particulier, il est décrit (non revendiqué) un procédé de production de chondroïtine dans une cellule génétiquement modifiée, le procédé comprenant :

1) l'obtention d'une cellule de la souche K12 *d'Escherichia coli,* comprenant au moins :

- un gène codant pour une glucuronyl transférase de mammifère, et les éléments permettant l'expression dudit gène dans ladite cellule,
- un gène codant pour une chondroïtine synthase bactérienne, et les éléments permettant l'expression dudit gène dans ladite cellule,
- un gène codant pour une béta-galactoside perméase, et les éléments permettant l'expression dudit gène dans ladite cellule,

2) la culture de ladite cellule en présence de béta-galactoside exogène et dans des conditions compatibles avec la production de chondroïtine par la cellule, et
3) la purification par chromatographie de la chondroïtine produite à l'étape 2).

Selon un autre aspect, l'invention a pour objet l'utilisation d'une cellule de *Escherichia coli* comprenant au moins les gènes *glcA-T, kfoC, kfiD* et *wbpP* pour la production de chondroïtine.

Selon un autre aspect, l'invention a pour objet l'utilisation d'une cellule de *Escherichia coli* comprenant au moins les gènes *glcA-T, kfiA, kfib, kfiC et kfiD* pour la production d'héparosane.

Il est également décrit (non revendiqué, ne faisant pas part de l'invention):

- l'utilisation de chondroïtine obtenue par la mise en œuvre d'un procédé selon l'invention pour la synthèse de chondroïtine sulfate.
- l'utilisation d'héparosane obtenu par la mise en œuvre d'un procédé selon l'invention pour la synthèse d'héparosane sulfate.
- l'utilisation de GAG obtenu par la mise en œuvre d'un procédé l'invention pour la préparation d'un médicament, pour la régénération ou l'ingénierie de tissus ou pour la réalisation d'un test de diagnostic. Plus particulièrement, l'utilisation de la chondroïtine obtenue par la mise en œuvre d'un procédé selon l'invention pour la préparation d'un médicament destiné au traitement d'une maladie choisie parmi : le cancer, l'osthéoarthrite et l'inflammation, ou pour un médicament destiné à être utilisé dans la régénération ou l'ingénierie tissulaire. Selon un autre aspect particulier, l'utilisation de l'héparosane obtenu par la mise en œuvre d'un procédé selon l'invention pour la préparation d'un médicament destiné à la prévention d'un accident thrombo-embolique ou destiné à être utilisé en tant qu'agent anti-coagulant.

- l'utilisation d'un GAG obtenu par la mise en œuvre d'un procédé selon l'invention, de préférence la chondroïtine ou l'héparosane, pour la préparation d'une composition alimentaire ou complément nutritionnel ou pour la préparation d'un produit cosmétique.
- l'utilisation de la chondroïtine obtenue par la mise en œuvre d'un procédé selon l'invention pour la préparation d'un complément nutritionnel.

D'autres caractéristiques et avantages de l'invention apparaissent dans les exemples et figures suivants.

LEGENDES DES FIGURES

**Figure 1:** Schéma synthétique de l'ingénierie métabolique des cellules génétiquement modifiées. Après l'entrée d'un accepteur dans la bactérie : expression hétérologue de glycosyltransférase d'intérêt (étape 2), accumulation des nucléotides sucres, substrats des glycosyltransférases (étape 3).

**Figure 2:** Schéma de la stratégie de synthèse de chondroïtine chez *E. coli* à partir de lactose exogène (Lac), avec galactose (Gal), glucose (Glc), Uridine Di Phosphate (UDP), acide glucuronique (GlcA), N-acetyl galactosamine (GalNAc), et

- Uridine-Di-Phosphate-glucose (UDP-Glc),
- Uridine-Di-Phosphate-acide glucuronique (UDP-GlcA),
- Uridine-Di-Phosphate- N-acetyl galactosamine (UDP-GalNac),
- Uridine-Di-Phosphate- N-acetyl glucosamine (UDP-GlcNac),
- gène de *E. coli* codant pour une GlcNAc transférase (*kfiA*)
- gène de *E. coli* codant pour une glcA transférase (*kfiC*),
- le gène de *E. coli* codant pour une UDP-Glucose 6 deshydrogénase (*kfiD*)
- le gène codant pour UDP-N-acétyl-glucosamine épimérase de *Pseudomonas aeroginosa.* (wbpP)
- gène codant pour la glucuronyl transférase (*glcA-T*)
- gène codant pour l'enzyme utilisant l'UDP-GlcA pour la synthèse de l'acide colanique (*wcaJ*)
- gène de *E. coli* codant pour la chondroïtine polymérase (*kfoC*)

**Figure 3:** Mesure des acides uroniques (barres noires) et des osamines (barres blanches) par dosages colorimétriques dans les fractions intracellulaires des souches recombinantes, avec de haut en bas KCB2, KCB1 et SBP9-24. La quantité en acides uroniques (UA) et en osamines est indiquée en g/L.

**Figure 4:** Mesure des acides uroniques et des osamines par dosages colorimétriques dans les fractions intra et extracellulaires des souches recombinantes, avec de haut en bas : fraction extracellulaire de KCO3, KCO1 et KCB2, fraction intracellulaire de KCO3, KCO1 et KCB2. La quantité en acides uroniques (UA, barres noires) et en osamines (barres blanches) est indiquée en g/L.

**Figures 5A et 5B:** Spectres issus de l'analyse en GPC de la chondroïtine produite par les souches recombinantes. Figure 5A : Analyse de la chondroïtine produite par la souche recombinante KCB2 (taille mesurée moyenne : 17.970 Da, polydispersité : 1,35). Figure 5B : Analyse de la chondroïtine produite par la souche recombinante KCO1 (taille mesurée moyenne : 73.510 Da, polydispersité : 1,42).

**Figures 6A à 6C:** Spectres issus de l'analyse en chromatographie d'exclusion stérique « Multiangle light scattering » (SEC-MALS) des fractions SL (Figure 6A), AL1 (Figure 6B) et AL2 (Figure 6C) obtenues lors de la production d'héparosane.

**Figure 7 :** Spectres issus de l'analyse en GPC (SEC-MALS) de la fraction héparosane produite dans la souche KHB3 à partir d'allyl-lactose. Pic 1, agrégats ; pic 2, Mw 52740 Da, Mw/Mn 1,17 ; pic 3, Mw 22346 Da, Mw/Mn 1,09.

EXEMPLES

**Exemple 1 : Ingénierie de la glycosylation chez Escherichia coli**

La souche K12 *d'E. coli* (appelée DJ) utilisée pour la production de chondroïtine à partir de lactose exogène est décrite dans Yavuz et al. (2008), cette souche est une souche *d'E. coli* DH1 (référence ATCC : 68153) modifiée comme indiqué dans la publication de Yavuz et al. La souche K12 utilisée est transformée par un plasmide possédant le gène *kfoC* de *E.coli* K4. L'enzyme kfoC utilise de l'UDP-GalNac qui est apporté par l'expression de *wbpP,* gène codant pour une UDP-N-acétyl-glucosamine épimérase de *Pseudomonas aeruginosa.* Le gène *wbpP* de *Pseudomonas aeruginosa,* codant pour l'UPD-Glc épimérase, a également été introduit dans la bactérie. Pour la synthèse du glucuronyl-lactose, l'enzyme GlcA-T permet l'ajout d'un acide glucuronique (GlcA) sur le lactose à partir d'UDP-GlcA. Le gène codant pour la glucuronyltransférase de cerveau de rat (GlcA-T) est également introduit dans la bactérie. Il est important d'avoir une accumulation d'UDP-GlcA en amont. Pour ce faire, la souche est transformée avec un plasmide contenant le gène *kfiD.* Ce gène code pour une UDP-glucose déshydrogénase présente chez K5 permettant la synthèse d'UDP-GlcA à partir d'UDP-Glc. De plus, la souche K12 a été rendue *LacZ-* et *wcaJ-* par délétions. Le gène *lacZ* fait partie de l'opéron lactose, il code pour une béta-galactosidase qui hydrolyse le lactose en galactose et glucose. Son inactivation permet d'empêcher la dégradation du lactose et augmente donc sa disponibilité pour la synthèse métabolique du produit d'intérêt. Le gène *wcaJ* code, quant à lui, pour une enzyme utilisant de l'UDP-GlcA dans la synthèse d'acide colanique. Son inactivation permet, dans le cas présent, une accumulation d'UDP-GlcA. De plus, l'acide colanique étant un oligosaccharide acide, il peut interférer lors des purifications de la chondroïtine, le fait d'inhiber sa synthèse est donc d'autant plus bénéfique. La souche K12 *lacZ-, wcaJ-* est appelée DJ.

Les différentes souches étudiées sont SBP9-24, KCB1 et KCB2 (Tableau 1).

Tableau 1 : Caractéristiques des souches étudiées

| Souche | Hôte | Plasmides presents | Résistances | Commentaires |
|---|---|---|---|---|
| SBP9-24 | DJ | pACT3-GlcA-T, pBS-kfoC, pBBR-kfiD, pWKS- wbpP | KATC | Souche de référence, 4 plasmides présents |
| KCB1 | DJ | pBBR-kfiD, pBS-kfoC-GlcA-T, pWKS- wbpP | KAT | 3 plasmides présents, gène GlcA-T inséré dans un plasmide à grand nombre de copies |
| KCB2 | DJ | pBBR-GlcA-T-kfiD, pBC-kfoC, pWKS- wbpP | KAT | 3 plasmides présents, GlcA-T inséré dans un plasmide à nombre moyen de copies |
| KCO1 | DJ | pBBR-kfiD, pBC-kfoC, pWKS- wbpP | KAT | Contrôle sans GlcA-T |
| KCO3 | DJ | pBBR-GlcA-T- kfiD, pBS-vide, pWKS- wbpP | KAT | Contrôle sans KfoC |

La souche SBP9-24 est considérée comme la construction de référence avec 4 plasmides, chacun portant un gène d'intérêt. KCB1 et KCB2 ne contiennent que 3 plasmides et se différencient par le plasmide qui porte 2 gènes (pBS-kfoC-GlcA-T pour KCB1 et pBBR-GlcA-T-kfiD pour KCB2), leur différence étant que KCB2 possède le gène GlcA-T dans un plasmide au taux d'expression inférieur à celui que possède KCB1. Deux souches contrôles sont faites : KCO1 et KCO3, afin d'observer l'impact de l'expression de GlcA-T (KCOl) et de KfoC (KC03) sur la production de chondroïtine. La souche BL21 *lacZ-, wcaJ-* de E. coli et transformée par les plasmides : pBBR-*glcAT-kfiD;* pBS-*kfoC ;* pWKS-*wbpP*, est également utilisée.

**Exemple 2 : Production de chondroïtine dans E. coli à partir de lactose exogène**

Les transformations de *E. coli* K12 sont effectuées à partir de la souche K12 « DJ » qui est *lacZ-* et *wcaJ-.* Les plasmides (200 ng pour pBBR et pWKS, 100 ng pour pBS) sont ajoutés dans 100 \uL de bactéries compétentes. Après 15 min dans la glace et un choc thermique de 45 secondes à 42°C, 400p.L de milieu SOC sont ajoutés et le tout est incubé 1 h à 37°C. Après concentration dans 100 microL de surnageant, le mélange est étalé sur boîte LBA + glucose (0,1% v/v) et les antibiotiques correspondants (0,1% v/v) et incubé à 30°C sur la nuit.

**Composition des milieux**

$KH_2PO_4$ (5 g/L), $NH4PO_4$ (5 g/L), Acide citrique (0,5 g/L), NaOH (0,66 g/L), KOH (1,65 g/L), TMS (7,5 mL/L), Glycérol (2,4 g/L). Le glucose (17,5 g/L) et le $MgS0_4$ (1 g/L) sont autoclavés séparément. La thiamine (10 mM) et les antibiotiques (35 mM) sont ajoutés après autoclave.

**Composition de l'alimentation**

Glycérol (750 g/L), $MgS0_4$ (18 g/L), TMS (37,50 mL/L). L'alimentation est délivrée à un débit de 9 mL/min pendant la deuxième phase puis à un débit de 6 mL/min pendant la troisième phase (cf déroulement des cultures).

**Déroulement des cultures**

Les cultures sont réalisées dans des fermenteurs Applikon de 3L avec un volume total de milieu de 1,5 L. Durant ces cultures, plusieurs paramètres sont contrôlés; l'oxygène à 40%, la température à 33 ou 28 °C suivant les phases de cultures et le pH à 6,80 avec de l'ammoniac à 30% (10). Le déroulement des cultures est divisé en plusieurs phases.

La première phase correspond à une phase de croissance avec l'apport d'une source carbonée, le glucose. Ce dernier permet une répression catabolique, ainsi l'opéron *lac,* utilisé comme promoteur dans les plasmides apportant les gènes d'intérêt, est inhibé. Cela permet d'éviter toute production avant l'induction est ainsi de favoriser la croissance des bactéries. Durant cette étape, la température de la culture est maintenue à 33°C. Cette étape est terminée lorsque tout le glucose ainsi que le glycérol présent dans le milieu de culture initial ont été consommés.

La deuxième phase est une phase d'induction avec l'apport d'alimentation contenant du glycérol comme source carbonée en remplacement du glucose du milieu initial. L'alimentation est administrée à un débit de 9 mL/min. Après un test de réaction de la culture à l'alimentation (arrêt de l'apport en alimentation et observation d'une augmentation de l'oxygène), la température de la culture est diminuée à 28 °C. Après un second test de réponse à l'alimentation à cette température, l'induction de l'expression des gènes d'intérêt a été faite par injection d'IPTG à 50 mg/L. A ce moment, le précurseur exogène nécessaire à la chaîne de synthèse, le lactose, est également apporté dans le milieu à une concentration de 1,6 g/L.

La troisième et dernière phase est entamée 5 h après l'induction par la diminution du débit d'alimentation (6 mL/min) de manière à ralentir la croissance bactérienne tout en continuant de fournir l'apport d'énergie nécessaire à la production du polysaccharide d'intérêt.

Durant les cultures, des prélèvements d' 1,5 mL sont faits 1 h après l'induction (1 h), le lendemain de l'induction (24 h), le troisième jour une heure après la seconde injection de lactose (48 h) et le dernier jour avant l'arrêt des cultures (72 h). Après centrifugation 5 min à 13000g, le surnageant est mis à part (fraction extracellulaire) alors que le culot est repris dans 1 mL d'eau distillée puis mis à 100°c au bain-marie pendant 10 min. Après resuspension et centrifugation 3 min à 13000g, le culot contient les débris bactériens et le surnageant correspond à la fraction intracellulaire. Les fractions issues de ces prélèvement sont analysées sur couche mince de silice (CCM) par migration dans un tampon n-butanol / acide acétique / eau (2:1:1) pendant 30 minutes. Le standard utilisé lors de ces CCM est un mélange GlcA-Lac / lactose. La révélation des CCM est faite avec un mélange acétate d'éthyle (100 mL), diphénylamine (2 g), aniline (2 mL), acide phosphorique 85% (10 mL) et acide chlorhydrique (1 mL).

Après l'arrêt des fermenteurs, la culture est récupérée puis centrifugée 20 min à 7000 rpm pour séparer les bactéries du milieu extracellulaire. Les culots bactériens, repris dans 500 mL d'H2O, sont soumis à un traitement thermique (20 min à l'autoclave à 100 °C) de manière à lyser les bactéries. Pour finir, les culots sont resuspendus et broyés puis centrifugés 20 min à 7000 rpm de manière à éliminer les débris bactériens et conserver uniquement le surnageant qui correspond à la fraction intracellulaire des bactéries. Les surnageants récupérés suite à la première centrifugation correspondent aux fractions extracellulaires.

**Purification et analyse des sucres**

Dans un premier temps, le pH des fractions est ajusté à 3,5 par traitement à la résine échangeuse de cations (amberlite IR-120) ce qui permet de précipiter les protéines, les fractions sont centrifugées 20 min à 7000 rpm pour éliminer le précipité protéique et les surnageant ont vu leur pH ajusté à 6,5 par traitement à la résine échangeuse d'anions (Dowex 66). Dans un second temps, 500 mL des fractions sont soumis à une précipitation à l'éthanol (3 volumes d'éthanol à 96% pour 1 volume de fraction) de manière à précipiter la chondroïtine. Après 3h à 4°C, le mélange est centrifugé 20 min à 7000 rpm et le culot solubilisé dans 80 mL d'eau.

Les fractions ainsi purifiées sont ensuite dosées en acide uronique (UA) et en osamine par dosage colorimétrique, respectivement par le procédé Blumenkrantz (1973) avec comme standard de l'acide glucuronique (GlcA) et celle de Morgan (1955) avec comme standard du N-acétyl galactosamine (GalNAc). Ces dosages ont permis d'obtenir une concentration théorique en chondroïtine grâce aux relations suivantes :

$$" Q_{\text{chondroïtine}} = (Q_{UA} / M_{qIca}) \, X \, (M_{QalNAc} - 18) + Q_{UA} "$$

$$« Q_{\text{chondroïtine}} = (Q_{\text{osamines}} / M_{GalNAc}) \, X \, (M_{GlcA} - 18) + Q_{\text{osamines}} »$$

Les produits ainsi obtenus après purification ont été congelés à l'azote liquide puis lyophilisés de manière à obtenir une masse sèche pesée et utilisée pour la caractérisation du produit.

Les différents produits de culture sont analysés en chromatographie de perméation de gel (GPC-SEC). La GPC-SEC est réalisée sur colonnes Shodex 802/803 éluées avec de nitrate de sodium (NaN03) 0,1 M à un débit de 0,5 mL/min. La détection est triple avec un réfractomètre Waters, un détecteur MALS (Multi Angle Light Scaterring) 18 angles (Wyatt) et un dn/dC de 0,142 (W02012/089777). Les données sont traitées avec le logiciel Astra (Wyatt).

**Résultats**

**Comparaison des différentes souches bactériennes recombinantes**

Plusieurs souches recombinantes sont comparées : SBP9-24, KCB1 et KCB2, afin de sélectionner la souche la plus intéressante en termes de qualité et de quantité pour la production du polysaccharide d'intérêt. Pour ce faire, il a été décidé dans un premier temps de comparer ces souches sur la synthèse de l'accepteur chimérique (GlcA-Lac).

Le lactose doit être correctement internalisé dans la bactérie pour permettre une accumulation suffisante en intracellulaire de GlcA-Lac. Ce dernier pourra alors être reconnu en tant qu'accepteur par KfoC. Ainsi durant les cultures des différentes souches recombinantes, plusieurs prélèvements ont été faits afin d'évaluer par CCM les concentrations et la répartition du lactose et du GlcA-Lac.

Il est possible de différencier les souches sur certains points. Tout d'abord, pour la vitesse de consommation du lactose, les souches à trois plasmides KCB1 et KCB2 ont totalement consommé le lactose du 1er jour après 24 à 48h ce qui n'est pas le cas de SBP9-24. Au vu de ce résultat, il a été décidé de procéder à une seconde injection de lactose (1,6 g/L) au bout de 48h, ce sera également le cas pour toutes les autres cultures. Ce constat s'accorde avec un deuxième qui est que le lactose est beaucoup plus rapidement internalisé par les souches KCB 1 et 2 que par la souche SBP9-24 où l'on observe plus longtemps du lactose en extracellulaire après une injection. Dans le cas des 3 souches, on observe que le lactose est métabolisé en GlcA-Lac, signe que le début de la synthèse métabolique a lieu. Il semble cependant que les souches à trois plasmides soient plus performantes au niveau de la production de GlcA-Lac (forte vitesse de consommation en lactose, présence plus importante en GlcA-Lac en fin de culture).

Dans un second temps, les souches sont comparées sur la production de chondroïtine elle- même, par le biais de dosages colorimétriques des acides uroniques et des osamines, permettant de quantifier la production de chondroïtine pour chaque souche.

Une production accrue d'acides uroniques et d'osamines dans les souches à trois plasmides est observée (Figure 3). Une des raisons pouvant expliquer cette différence est le fait que la souche SBP9-24 pousse plus difficilement que les deux souches à trois plasmides. Entre KCB1 et KCB2, il semble que KCB2 produise davantage, signe que l'expression de GlcA-T n'a pas besoin d'être très forte puisque que cette enzyme est codée dans un plasmide à nombre moyen de copies (pBBR) chez KCB2 et non avec un plasmide à fort nombre de copies (pBS) dans KCB1.

Il semble donc que la souche recombinante KCB2 soit la plus productive en chondroïtine à partir de l'accepteur chimérique, C'est donc cette souche qui a été choisie pour faire la production et la caractérisation de la chondroïtine.

**Analyse de la production de chondroïtine par la souche KCB2**

Il est important de montrer que la production de chondroïtine chez KCB2 se fait à partir de l'accepteur chimérique, le glucuronyl-lactose. Pour cela, une souche contrôle, KCO1, est construite, elle ne possède pas le gène codant pour GlcA-T. Ainsi, ce contrôle négatif ne peut transformer le lactose en GlcA-Lac. Cela donne une indication sur la production de chondroïtine lorsque le glucuronyl-lactose n'est pas présent.

L'absence de glucuronyl-lactose chez KCO1 entraîne une baisse de la concentration en acide uronique et en osamine (Figure 4) donc en chondroïtine (0,5 g/L) dans la fraction intracellulaire. Cette quantité est environ trois fois inférieure à celle produite chez KCB2 (1,7 g/L). Ainsi, il apparaît qu'en l'absence de glucuronyl-lactose, la synthèse de chondroïtine s'effectue quand même, nécessairement à partir d'un accepteur endogène.

La production de chondroïtine dans la souche recombinante KCB2 est également comparée à celle dans un autre contrôle : la souche KCO3. Cette souche est dépourvue du gène codant pour KfoC. Son absence dans la souche KCO3 résulte en la quasi absence d'acide uronique et d'osamine que ce soit dans la fraction homologue ou en extracellulaire (Figure 4). A partir de la CCM issue des prélèvements de culture, il est observé que la production de glucuronyl-lactose a bien lieu. Ainsi, KfoC est indispensable à la synthèse de chondroïtine et cela même si l'accepteur chimérique est présent.

**Caractérisation et analyse de la chondroïtine**

Il a été montré précédemment qu'en l'absence de glucuronyl-lactose, la synthèse de chondroïtine a lieu à partir d'un accepteur endogène. Cela correspond à des résultats en synthèse *in vivo* montrés dans diverses publications. Cependant, l'analyse en GPC donne une taille de chondroïtine chez KCB2 (produite à partir de glucuronyl-lactose) de 17,5 kDa (Figure 5A) alors que les publications font rapport d'une taille de chondroïtine synthétisée (chez K4 ou K5) aux alentours de 100 à 600 kDa. On peut dès lors se demander si la faible taille obtenue chez KCB2 est due aux propriétés de la souche K12 ou au fait d'initier la synthèse par un accepteur différent. Pour répondre à cette question, une analyse en GPC est également menée sur les produits purifiées de KCO1 (souche contrôle sans GlcA-T) et la taille mesurée de la chondroïtine produite est d'environ 73 kDa (Figure 5B). Ainsi, la présence de glucuronyl-lactose, en tant que précurseur de la synthèse, entraîne la production d'une chondroïtine différente, plus petite de celle produite à partir de l'accepteur endogène.

Un autre fait remarquable est qu'en présence de glucuronyl-lactose, la chondroïtine de 73 kDa est complètement absente (taille entre 15 et 20 kDa, polydispersité de 1,35) (Figure 5A). Deux hypothèses peuvent être formulées pour expliquer ce constat. La première est que la production de glucuronyl-lactose résulte en une plus grande présence de substrat pour KfoC que lorsque seul l'accepteur endogène est utilisé. Ainsi, l'enzyme KfoC recommence plus souvent la synthèse à partir d'oligosaccharides et de précurseur au lieu de poursuivre l'élongation de polysaccharides. Il en résulte ainsi une baisse de la taille moyenne de la chondroïtine produite.

La seconde hypothèse est que l'environnement membranaire stabilise le couplage enzyme- chondroïtine au profit de la polymérisation du polysaccharide. En effet, à l'état sauvage, la synthèse de chondroïtine a lieu au niveau de l'espace péri-membranaire, l'accepteur endogène étant membranaire. A ce niveau, KfoC a l'appui de complexe membranaire pour la synthèse de chondroïtine dont les rôles sont mal connus (Whitfield, 2006). Le glucuronyl-lactose étant cytoplasmique, la synthèse, dans le cas de cette étude, a alors lieu loin de la membrane. Ce positionnement cytoplasmique de la synthèse a pu avoir un impact sur l'élongation de la chondroïtine.

La production de chondroïtine dans une souche recombinante non pathogène de *E. coli* est donc intéressante car elle apporte, par rapport aux autres modes de production, une facilité de manipulation. Sur le plan qualitatif, la chondroïtine obtenue est d'une taille plus faible que celle obtenu par d'autres moyens (15 kDa contre une centaine de kDa en culture et 20 kDa *in vitro*). Ce mode de production est aussi avantageux au niveau de la quantité produite qui atteint les 1,7 g/L alors que les plus hautes productions en fermenteur chez *E. coli* K4 sont rapportées comme ayant une production de 1,4 g/L (Schiraldi *et al.,* 2010).

La chondroïtine produite a été analysée par RMN. La structure du motif de repétition [GlcAb-3GalNAcb-4] est confirmée par analyse RMN du $^{1}H$ et du $^{13}C$, dont les résultats sont conformes à ceux publiés dans la littérature pour le polysaccharide K4 défructosylé et la chondroïtine de Pasteurella multocida. Le spectre RMN du proton montre des signaux à 4.55, 3.43, 3.64 ppm qui correspondent aux H-1, H-3, et H-2 du résidu Glcp-UA. Les signaux à 4.18 et 2.08 ppm correspondent aux H-4 et H(Ac) du résidu Galp-NAc. La RMN du carbone montre les assignements caractéristiques du groupement N-acétamido à 23.91 et 176.22 ppm. On retrouve aussi l'assignement caractéristique du C-6 du résidu Glcp-UA à 175.61 ppm.

**Exemple 3 : Production de chondroïtine dans E. coli à partir de béta-methylgalactoside exogène**

Les souches bactériennes sont préparées et cultivées selon le protocole décrit dans l'exemple 2, à l'exception du fait que le béta-galactoside exogène consiste en du béta-methylgalactoside, ajouté à raison de 4,2 grammes dans le milieu de culture. La quantité de béta-méthylgalactoside ajouté au milieu est identique à la quantité de lactose ajouté dans l'exemple 2, en tenant compte des masses molaires différentes des deux produits.

La fraction chondroïtine produite présente une taille de 37-61 kDa, c'est-à-dire une taille inférieure à celle de la chondroïtine obtenue en absence d'accepteur exogène, mais supérieure à celle obtenue en présence de lactose.

**Exemple 4 : Production d'héparosane par une souche d'Escherichia coli K12 en présence de glucuronyl-lactose intracellulaire.**

La production d'héparosane recombinant représente un enjeu particulier car ils peuvent être utilisés comme précurseurs de l'héparine, en particulier s'ils sont de petite taille d'environ 15-20 kDa (Manzoni *et al.,* 1996). L'héparosane est formé d'un enchainement de [-4)-$\beta$-GlcA-(1-4)-$\alpha$-GlcNAc-(1-]. Sa voie de biosynthèse est semblable à celle du polysaccharide K4 et les gènes associés à l'exportation sont les mêmes (Whitfield, 2006).

Une souche d'E coli K12 a été modifiée pour produire de l'héparosane (Barreteau *et al.,* 2012). Cette souche exprime les gènes *kfiABCD de E. coli* (X77617 du 23 octobre 2008), dont la présence est nécessaire et suffisante pour produire de l'héparosane en intracellulaire, d'une taille approximative de 80 kDa. Cette souche a maintenant été transformée par un plasmide exprimant la glucuronyltransférase de souris permettant la synthèse intracellulaire de glucuronyl-lactose en présence de lactose. Pour suivre le résultat de cette étude, un premier indice attendu de la prise en charge du glucuronyl-lactose par les polymérases est la synthèse d'un polysaccharide de plus petite taille, si on s'en réfère aux résultats obtenus avec la chondroïtine pour lesquels c'est effectivement le cas.

La même souche a été cultivée dans des fermenteurs de 2 litres en présence (7,5g de lactose ajoutés dans l'alimentation sur 3 jours).et en absence de lactose extracellulaire.

Les résultats sont les suivants :
Dosage des acides uroniques de la fraction précipitée à l'éthanol :
Sans ajout de lactose : 825 mg total (environ 0,8 g/L héparosane)

Avec ajout de lactose : 444 mg total (environ 0,45 g/L héparosane)

Analyse de la taille des héparosanes recombinants :
Les fractions ont été analysées en SEC-MAL pour l'obtention de la taille et de la dispersité des héparosanes produits. Cependant, la présence importante d'agrégats a nécessité une étape ultérieure de purification par chromatographie d'échange d'ions.

La fraction « sans lactose » a été passée sur DEAE-A25 et une fraction retenue représentant 40% du total déposé a appelée SL a été récupérée.

La fraction « avec lactose » a été passée sur DEAE-A25 et une fraction retenue appelée AL1 représentant seulement 20% du total déposé a été récupérée, cet écart de rendement pouvant être dû à la nature des héparosanes produits. Il a été décidé d'utiliser un échangeur plus fort de type QAE-A25. Ceci a permis la récupération d'une deuxième fraction appelée AL2, représentant 30% du total de la fraction initiale.Les fractions SL, AL1 et AL2 ont été analysées en SEC-MALS sans formation d'agrégats (Figures 6A à 6C). Les caractéristiques des pics analysés sont les suivantes :

Tableau 2 : Caractéristiques des héparosanes produits

| | | **Pic 1** | **Pic 2** |
|---|---|---|---|
| **SL** | % total (RI detection) | 67 | 33 |
| | Mw (Da) | 7,57 x $10^4$ | 2,05 x $10^4$ |
| | Mz (Da) | 1,06 x $10^5$ | 2,26 x $10^4$ |
| | Polydispersité (Mw/Mn) | 1,4 | 1,1 |
| **AL1** | % | 44 | 68 |
| | Mw (Da) | 5,49 x $10^4$ | 1,87 x $10^4$ |
| | Mz (Da) | 6,43 x $10^4$ | 2,17 x $10^4$ |
| | Polydispersité (Mw/Mn) | 1,4 | 1,3 |
| **AL2** | % | 49 | 51 |
| | Mw (Da) | 6,94 x $10^4$ | 2,72 x $10^4$ |
| | Mz (Da) | 7,35x $10^4$ | 2,89 x $10^4$ |
| | Polydispersité (Mw/Mn) | 1,4 | 1,06 |

La fraction SL est essentiellement composée d'un polymère de taille déjà observée (Barreteau *et al.,* 2012). Cependant, elle possède aussi une fraction de taille plus faible qui représente environ 30% du total. Les fractions AL1 et AL2 se distinguent par un enrichissement de la plus petite fraction (pic 2), qui représente au moins 50-70 % du total basé sur une quantification en réfractométrie. La production intracellulaire dans une souche recombinante d'E coli K12 capable de synthétiser de l'héparosane recombinant induit une modification de la quantité et des caractéristiques des héparosanes produits. Il en résulte un enrichissement d'un héparosane de petite taille d'environ 20 kDa.

**Exemple 5 : Production d'héparosane dans une souche Escherichia coli K12 en présence de glucuronyl-lactose intracellulaire.**

Les gènes spécifiques pour la synthèse de l'héparosane d'E coli appartiennent au cluster de l'opéron K5 et sont : *kfiA,* codant pour une GlcNAc transférase; *kfiB,* dont le rôle est inconnu; *kfiC,* codant pour une glcA transférase; *kfiD,* codant pour une UDP-Glc deshydrogénase.

Le but de cette étude est l'ingénierie métabolique de E coli K12 afin de permettre une synthèse d'héparosane à partir d'un accepteur cytoplasmique exogène béta-glucuronylé provenant de l'action de la GlcA-T de souris (codé par le gène *glcAT)* sur un béta-galactoside. Plusieurs souches sont réalisées, décrites dans le tableau ci-dessous. La souche HB5 est décrite dans Barreteau *et al.* (2012).

Tableau 3 : Caractéristiques des souches réalisées

| Souche | Hôte | Plasmides | | | | | |
|---|---|---|---|---|---|---|---|
| | | pBBR-kfiAB Tet, low copy | pSU-kfiCD Cm, low copy | pBBR-GlcAT-kfiD Tet, low copy | pBBR-kfiABCD Tet, low copy | pBS-kfiAC Amp, high copy | pSU-GlcAT Cm, low copy |
| HB5 | DJ | + | + | | - | - | - |
| KHB1 | DJ | - | - | + | - | + | - |
| KHB2 | DJ | - | - | - | + | - | + |
| KHB3 | DJ | - | - | - | + | + | + |

Une première souche KHB1 est réalisée, exprimant les gènes *kfiA* et *kfiC* codant pour les 2 glycosyltransférases impliquées dans la synthèse de l'héparosane dans un plasmide pBluescript (pBS) à grand nombre de copies, aux côté des gènes *kfiD* (synthèse d'UDP-GlcA) et *glcAT* (glucuronyltransférase de souris). Cette souche n'exprime pas KfiB, dans la mesure où il a été montré que cette protéine favorise la fixation de KfiC à la membrane, où se situe l'accepteur endogène membranaire (Hodson *et al.,* 2000). Le but ici étant de réaliser la synthèse d'héparosane à partir du glucuronyl-lactose, on peut penser que cette association à la membrane ne soit plus nécessaire, voire néfaste car la localisation de l'accepteur endogène est également membranaire. Deux cultures sont menées avec ou sans lactose ajouté. Le lactose est ajouté à raison de 7,5g (pour 2 1 de culture) de façon continue pendant les 3 jours d'alimentation après induction. Les résultats sont obtenus après précipitation éthanolique de la fraction intracellulaire et dosage des acides uroniques. Aucune synthèse d'héparosane n'a été obtenue, que ce soit en présence ou en absence de lactose (Tableau 4). Ces résultats corroborent néanmoins ceux de la publication de Hodson *et al.,* (2000), qui mentionne le rôle crucial de KfiB dans la stabilité du complexe enzymatique héparosane synthase.

Il est donc décidé d'exprimer l'ensemble des gènes *kfiABCD,* en y ajoutant l'expression de la glucuronyltransférase de souris. Pour ce faire, les 4 gènes *kfiABCD* sont clonés dans un même plasmide à faible copie, ceci afin de permettre la co-expression avec le gène *glcA-T* dans un deuxième plasmide pSU également à faible nombre de copies. La souche baptisée KHB2 est cultivée en présence et en absence de lactose. En absence de lactose, cette souche produit une quantité d'héparosane comparable à celle obtenue avec la souche HB5 précédemment publiée (Barreteau *et al.,* 2012). Ceci est cohérent avec le faite que ces 2 souches sont comparables y compris quant aux niveaux d'expression des 4 gènes *kfiABCD,* tous clonés dans des plasmides à faible nombre de copies. L'absence de lactose prive la bactérie de l'accepteur potentiel glucuronyl-lactose, ce qui explique les résultats similaires avec la souche de référence. En présence de lactose, il s'en suit une réduction de la production d'héparosane, mais le fait intéressant est que l'on observe un enrichissement de la fraction héparosane de petite taille, ce qui laisse présager que le complexe héparosane synthase a pris en charge le glucuronyl-lactose comme accepteur de polymérisation, dans la mesure où ce phénomène s'observe avec la synthèse de chondroïtine à partir du glucuronyl-lactose. Ces résultats montrent un faible rendement de production d'héparosane, et une fraction polysaccharidique obtenue composée de 2 fractions de tailles différentes.

Une nouvelle souche baptisée KHB3 est alors réalisée. Il s'agit en fait d'une souche pour laquelle le niveau d'expression des gènes *kfiA* et *kfiC* est augmenté, en comparaison des autres gènes, ceci afin d'augmenter le niveau des activités glycosyltransférases par rapport à l'expression de KfiB. Les résultats sont surprenants : en présence de lactose, la production d'héparosane est doublée par rapport à la souche de référence HB5, ce qui est tout à fait inédit. Ce résultat a été confirmé en cultivant cette même souche en présence de lactose-allyle. Sans lactose, il n'y a pas augmentation de la production d'héparosane, et ce, malgré la plus forte expression des enzymes héparosane synthase KfiA et KfiC. Egalement pour la chondroïtine, nous avions observé que l'ajout de lactose augmentait le rendement de production. Ceci laisse à penser que la concentration en accepteur endogène membranaire est un facteur limitant de la production de chondroïtine et d'héparosane dans la souche Escherichia coli K-12. La présence abondante d'un accepteur auxiliaire cytoplasmique lèverait cette limitation. La faible expression de KfiB suffit apparemment à stabiliser le complexe enzymatique KfiA/KfiC malgré la plus forte expression de ce dernier.

L'analyse en GPC a été réalisée sur la fraction préparée à partir d'allyl-lactose (Figure 7). Elle est composée de 2 espèces polysaccharidiques de taille 53 kDa et 22 kDa présentant une très faible polydispersité. Ces tailles sont inférieures à ce qui est produit normalement avec les gènes *kfi,* dans notre laboratoire et aussi dans la littérature et brevets existants. Ce phénomène est conforme à ce qui est observé avec la production de chondroïtine, où une réduction de taille en présence du glucuronyl-lactose est également observée.

Tableau 4 : Quantité d'héparosane produit (exprimée en g/L de culture).

| Souche | Sans lactose | Avec lactose | Avec lactose allyle |
|---|---|---|---|
| HB5 | 1,2 | Non réalisé | Non réalisé |
| KHB1 | Non détecté | traces | Non réalisé |
| KHB2 | 0,9 | 0,45 | Non réalisé |
| KHB3 | 0,84 | 2,6 | 2,7 |

RÉFÉRENCES BIBLIOGRAPHIQUES

Cimini et al. (2010) Appl. Microbiol, and Biotechnol, 85(6):1779-1787.
Jin et al. (2011) Biosci. Biotechnol. Biochem., 75(7), 1283-1289.
Yamada and Sugahara (2008) Current Drug Discovery Technologies, 5, 289-301.
Zanfardino et al. (2010) Microbiol. Cell Factories, 9:34
Wang et al. (2011) Bioengineered Bugs, 2:1, 63-67 (January/February)
Schiraldi et al. (2010) Appl. Microbiol, and Biotechnol, 87:1209-1220.
Inoué et al. (1990) Gene, 96:23-28.
Yavuz et al. (2008) Glycobiology, 18(2):152-157.
Ninomiya et al. (2002) J. Biol. Chem., Jun 14;277(24):21567-75.
Priem et al. (2002) Glycobiology. Vol. 12 no. 4pp. 235-240.
Kràhulec et al. (2005) Molecular Biology, 30:129-134.
Kakuda et al. (2004) J. Biol. Chem., 279 (21), pp 22693-22703.
Whitfield (2006) Annual Review ofBiochemistry, 75:39-68.
Morgan et al. (1955) Biochem. Journal, 61(4):586-589.
Blumenkrantz et al. (1973) Analytical biochemistry, 54:484-489.
Manzoni et al. (1996) J. Bioactive and Compatible Polymers, Vol.11, p301
Barreteau et al. (2012) Carbohydrate Research, 1:360:19-24.
Hodson et al. (2000) J. Biol. Chem., 275(35): 27311-5.

## Revendications

1. Procédé de production de glycosaminoglycane (GAG) dans une cellule génétiquement modifiée, ledit procédé comprenant les étapes suivantes :

    a) l'obtention d'une cellule comprenant au moins

        • un gène exogène codant pour une enzyme de mammifère capable d'exercer une activité glucuronyl-transférase sur un substrat béta-galactoside, et les éléments permettant l'expression dudit gène dans ladite cellule et la synthèse de glucuronyl béta-galactoside,
        • un gène codant pour une enzyme capable de synthétiser ledit glycosaminoglycane à partir du glucuronyl béta-galactoside, ou les gènes codant pour les enzymes de la voie de synthèse dudit glycosaminoglycane à partir du glucuronyl béta-galactoside, et les éléments permettant l'expression dudit ou desdits gènes dans ladite cellule,
        • un gène codant pour une enzyme capable d'assurer l'internalisation par la cellule de béta-galactoside exogène, et les éléments permettant l'expression dudit gène dans ladite cellule,

    b) la culture de ladite cellule en présence de béta-galactoside exogène et dans des conditions compatibles avec la production dudit glycosaminoglycane par ladite cellule, dans lequel ladite cellule est une cellule de bactérie.

2. Procédé selon la revendication 1, dans lequel l'enzyme capable d'exercer une activité glucuronyl transférase sur un substrat béta-galactoside est la glucuronyl-transférase de souris.

3. Procédé selon l'une des revendications 1 à 2, **caractérisé en ce que** ladite cellule comprend en outre un gène codant pour une enzyme capable de synthétiser l'UDP-GlcA (Uridine-Di-Phosphate-acide glucuronique) à partir d'UDP-Glc (Uridine-Di-Phosphate-glucose), et les éléments permettant l'expression dudit gène dans ladite cellule.

4. Procédé selon la revendication 3, **caractérisé en ce que** l'enzyme capable de synthétiser l'UDP-GlcA (Uridine-Di-Phosphate-acide glucuronique) à partir d'UDP-Glc (Uridine-Di-Phosphate-glucose) est une UDP-glucose deshydrogénase, de préférence l'UDP-glucose déshydrogénase codée par le gène kfiD de *E. coli* K5.

5. Procédé selon l'une des revendications 1 à 4, **caractérisé en ce que** ladite cellule comprend en outre un gène codant pour une enzyme capable de synthétiser l'UDP-GalNAc (Uridine-Di-Phosphate- N-acetyl galactosamine), et les éléments permettant l'expression dudit gène dans ladite cellule.

6. Procédé selon l'une des revendications 1 à 5 **caractérisé en ce que** ladite bactérie est une bactérie de souche *Escherichia coli* non pathogène.

7. Procédé selon l'une des revendications 1 à 6, **caractérisé en ce que** ledit glycosaminoglycane est la chondroïtine.

8. Procédé de production de chondroïtine dans une cellule génétiquement modifiée selon la revendication 7, dans lequel l'enzyme capable de synthétiser la chondroïtine à partir de glucuronyl-béta-galactoside est la chondroïtine synthase bactérienne de la souche K4 *d'Escherichia coli* capable de synthétiser la chondroïtine à partir de glucuronyl-lactose.

9. Procédé de production de chondroïtine dans une cellule génétiquement modifiée selon l'une des revendications 7 ou 8, ledit procédé comprenant l'obtention d'une cellule bactérienne de souche *Escherichia coli,* de préférence une cellule de souche K12 *d'Escherichia coli,* ladite cellule bactérienne comprenant au moins :

    • un gène exogène codant pour une glucuronyl transférase de mammifère, et les éléments permettant l'expression dudit gène dans ladite cellule,
    • un gène exogène codant pour une chondroïtine synthase bactérienne, et les éléments permettant l'expression dudit gène dans ladite cellule,
    • un gène exogène codant pour une lactose perméase, et les éléments permettant l'expression dudit gène dans ladite cellule,
    • un gène exogène codant pour une UDP-glucose deshydrogénase, et
    • un gène exogène codant pour l'UDP-N-acetyl-glucosamine épimérase de *Pseudomonas aeroginosa,*

et les gènes *wcaJ* (enzyme de synthèse de l'acide colanique) et *LacZ* (β galactosidase) de ladite cellule bactérienne ayant été inactivés.

**10.** Procédé de production de chondroïtine dans une cellule génétiquement modifiée selon l'une des revendications 7 à 9, ledit procédé comprenant l'obtention d'une cellule bactérienne de type *Escherichia coli,* de préférence une cellule de la souche K12 *d'Escherichia coli,* ladite cellule comprenant au moins les gènes *glcA-T* (béta-1-3-glucuronyl transférase), *kfoC* (chondroïtine polymérase), *kfiD* (UDP-Glucose 6 deshydrogénase) et *wbpP* (UDP-N-acétyl-glucosamine épimérase).

**11.** Procédé selon l'une des revendications 1 à 6, **caractérisée en ce que** ledit glycosaminoglycane est l'héparosane.

**12.** Procédé de production d'héparosane dans une cellule génétiquement modifiée selon la revendication 11, ledit procédé comprenant l'obtention d'une cellule bactérienne de souche *Escherichia coli,* de préférence une cellule de la souche K12 *d'Escherichia coli,* ladite cellule comprenant au moins : a) le gène *glcA-T* (béta-1-3-glucuronyl transférase), et b) les gènes *kfiA* (GlcNAc transférase), *kfiB*, *kfiC* (glcA transférase) *et kfiD* (UDP-Glucose 6 deshydrogénase) *de E. coli* ou au moins un gène codant pour une protéine ayant une fonction homologue à celle d'une protéine codée par *kfiA, kfiB kfiC ou kfiD.*

**13.** Utilisation d'une cellule de *Escherichia coli* comprenant au moins les gènes *glcA-T* (béta-1-3-glucuronyl transférase), *kfoC* (chondroïtine polymérase), *kfiD* (UDP-Glucose 6 deshydrogénase) et *wbpP* (UDP-N-acétyl-glucosamine épimérase) pour la production de chondroïtine.

**14.** Utilisation d'une cellule de *Escherichia coli* comprenant au moins les gènes *glcA-T* (béta-1-3-glucuronyl transférase), *kfiA* (GlcNAc transférase), *kfiB kfiC* (glcA transférase) *et kfiD* (UDP-Glucose 6 deshydrogénase) pour la production d'héparosane.

**Patentansprüche**

**1.** Verfahren zur Herstellung von Glykosaminoglykan (GAG) in einer genetisch veränderten Zelle, wobei das Verfahren die folgenden Schritte umfasst:

a) beim Erhalt einer Zelle, die mindestens umfasst:

- ein exogenes Gen, das für ein Säugetierenzym codiert, das in der Lage ist, eine Glucuronyltransferaseaktivität auf einem Beta-Galactosid-Substrat auszuüben, und die Elemente, die die Expression des Gens in der Zelle und die Synthese von Glucuronyl-Beta-Galactosid ermöglichen,
- ein Gen, das für ein Enzym codiert, das in der Lage ist, das Glykosaminoglykan ausgehend von dem Glucuronyl-Beta-Galactosid zu synthetisieren, oder die Gene, die für die Enzyme des Synthesewegs des Glykosaminoglykans ausgehend von dem Glucuronyl-Beta-Galactosid codieren, und die Elemente, die die Expression des oder der Gene in der Zelle ermöglichen,
- ein Gen, das für ein Enzym codiert, das in der Lage ist, die Internalisierung durch die exogene Beta-Galaktosid-Zelle sicherzustellen, und die Elemente, die die Expression des Gens in der Zelle ermöglichen,

b) die Kultur der Zelle bei Vorhandensein von exogenem Beta-Galactosid und unter Bedingungen, die mit der Produktion des Glukosaminoglykans durch die Zelle verträglich sind, wobei die Zelle eine Bakterienzelle ist.

**2.** Verfahren nach Anspruch 1, wobei das Enzym, das in der Lage ist, eine Glucuronyl-Transferase-Aktivität auf einem Beta-Galaktosid-Substrat auszuüben, Glucuronyl-Transferase von Mäusen ist.

**3.** Verfahren nach einem der Ansprüche 1 bis 2, **dadurch gekennzeichnet, dass** die Zelle ferner ein Gen, das für ein Enzym codiert, das in der Lage ist, UDP-GlcA (Uridindiphosphat-Glucuronsäure) ausgehend von UDP-Glc (Uridindiphosphat-Glucose) zu synthetisieren, und die Elemente umfasst, die die Expression des Gens in der Zelle ermöglichen.

**4.** Verfahren nach Anspruch 3, **dadurch gekennzeichnet, dass** das Enzym, das in der Lage ist, UDP-GlcA (Uridindiphosphat-Glucuronsäure) ausgehend von UDP-Glc (Uridindiphosphat-Glucose) zu synthetisieren, eine UDP-Glucose-Dehydrogenase, vorzugsweise UDP-Glucose-Dehydrogenase, ist, die durch das Gen kfiD von *E. coli* K5 codiert wird.

5. Verfahren nach einem der Ansprüche 1 bis 4, **dadurch gekennzeichnet, dass** die Zelle ferner ein Gen umfasst, das für ein Enzym codiert, das in der Lage ist, UDP-GalNAc (Uridindiphosphat-N-Acetylgalactosamin) zu synthetisieren, und die Elemente umfasst, die die Expression des Gens in der Zelle ermöglichen.

6. Verfahren nach einem der Ansprüche 1 bis 5, **dadurch gekennzeichnet, dass** die Bakterie eine nicht pathogene Bakterie vom Stamm *Escherichia coli* ist.

7. Verfahren nach einem der Ansprüche 1 bis 6, **dadurch gekennzeichnet, dass** das Glykosamninoglykan Chondroitin ist.

8. Verfahren zur Herstellung von Chondroitin in einer genetisch veränderten Zelle nach Anspruch 7, wobei das Enzym, das in der Lage ist, das Chrondroitin ausgehend von Glucuronyl-Beta-Galactosid zu synthetisieren, die bakterielle Chrondroitinsynthase des Stamms K4 von *Escherichia coli* ist, die in der Lage ist, das Chondroitin ausgehend von Glucuronyl-Lactose zu synthetisieren.

9. Verfahren zur Herstellung von Chondroitin in einer genetisch veränderten Zelle nach einem der Ansprüche 7 oder 8, wobei das Verfahren den Erhalt einer Bakterienzelle vom Stamm *Escherichia coli,* vorzugsweise einer Zelle vom Stamm K12 von *Escherichia coli,* umfasst, wobei die Bakterienzelle mindestens umfasst:

   - ein exogenes Gen, das für eine Säugetier-Glucuronyltransferase codiert, und die Elemente, die die Expression des Gens in der Zelle ermöglichen,
   - ein exogenes Gen, das für eine bakterielle Chondroitinsynthase codiert, und die Elemente, die die Expression des Gens in der Zelle ermöglichen,
   - ein exogenes Gen, das für eine Lactosepermease codiert, und die Elemente, die die Expression des Gens in der Zelle ermöglichen,
   - ein exogenes Gen, das für eine UDP-Glucosedehydrogenase codiert, und
   - ein exogenes Gen, das für die UDP-N-Acetylglucosamid-Epimerase von *Pseudomonas aeruginosa* codiert, und die Gene *wcaJ* (Syntheseenzym der Colansäure) und *LacZ* (β-Galactosidase) der Bakterienzelle deaktiviert wurden.

10. Verfahren zur Herstellung von Chondroitin in einer genetisch veränderten Zelle nach einem der Ansprüche 7 bis 9, wobei das Verfahren den Erhalt einer Bakterienzelle vom Typ *Escherichia coli,* vorzugsweise einer Zelle des Stamms K12 von *Escherichia coli,* umfasst, wobei die Zelle mindestens die Gene *glcA-T* (Beta-1-3-Glucuronyltransferase), *kfoC* (Chondroitin-Polymerase), *kfiD* (UDP-Glucose-6-Dehydrogenase) und *wbpP* (UDP-N-Acetylglucosamin-Epimerase) umfasst.

11. Verfahren nach einem der Ansprüche 1 bis 6, **dadurch gekennzeichnet, dass** das Glykosamninoglykan Heparosan ist.

12. Verfahren zur Herstellung von Heparosan in einer genetisch veränderten Zelle nach Anspruch 11, wobei das Verfahren den Erhalt einer Bakterienzelle vom Stamm *Escherichia coli,* vorzugsweise einer Zelle vom Stamm K12 von *Escherichia coli,* umfasst, wobei die Zelle mindestens umfasst: a) das Gen *glcA-T* (Beta-1-3-Glucuronyltransferase) und b) die Gene *kfiA* (GlcNAc-Transferase), *kfiB, kfiC* (glcA-Transferase) und *kfiD* (UDP-Glucose-6-Dehydrogenase) von *E. coli* oder mindestens ein Gen, das für ein Protein codiert, das eine Funktion aufweist, die homolog zu derjenigen eines durch *kfiA, kfiB, kfiC* oder *kfiD* codierten Proteins ist.

13. Verwendung einer *Escherichia coli* Zelle, die mindestens die Gene *glcA-T* (Beta-1-3-Glucuronyltransferase), *kfoC* (Chondroitin-Polymerase), *kfiD* (UDP-Glucose-6-Dehydrogenase) und *wbpP* (UDP-N-Acetylglucosamin-Epimerase) umfasst, zur Herstellung von Chondroitin.

14. Verwendung einer *Escherichia coli* Zelle, die mindestens die Gene *glcA-T* (Beta-1-3-Glucuronyltransferase), *kfiA* (GlcNAc-Transferase), *kfiB, kfiC* (glcA-Transferase) *und kfiD* (UDP-Glucose-6-Dehydrogenase) umfasst, zur Herstellung von Heparosan.

## Claims

1. A process for producing glycosaminoglycan (GAG) in a genetically modified cell, said process comprising the following steps:

   a) obtaining a cell comprising at least

      • an exogenous gene encoding a mammalian enzyme capable of exerting glucuronyl transferase activity on a beta-galactoside substrate, and the elements enabling the expression of said gene in said cell and the synthesis of glucuronyl beta-galactoside,
      • a gene encoding an enzyme capable of synthesizing said glycosaminoglycan from glucuronyl beta-galactoside, or the genes encoding the enzymes of the synthetic pathway of said glycosaminoglycan from glucuronyl beta-galactoside, and the elements enabling the expression of said gene(s) in said cell,
      • a gene encoding an enzyme capable of ensuring the internalization of exogenous beta-galactoside by the cell, and the elements enabling the expression of said gene in said cell,

   b) culturing said cell in the presence of exogenous beta-galactoside and under conditions compatible with the production of said glycosaminoglycan by said cell, wherein said cell is a bacterial cell.

2. The process as claimed in claim 1, wherein the enzyme capable of exerting glucuronyl transferase activity on a beta-galactoside substrate is mouse glucuronyl transferase.

3. The process as claimed in one of claims 1 to 2, **characterized in that** said cell further comprises a gene encoding an enzyme capable of synthesizing UDP-GlcA (uridine-diphosphate-glucuronic acid) from UDP-Glc (uridine-diphosphate-glucose), and the elements enabling the expression of said gene in said cell.

4. The process as claimed in claim 3, **characterized in that** the enzyme capable of synthesizing UDP-GlcA (uridine-diphosphate-glucuronic acid) from UDP-Glc (uridine-diphosphate-glucose) is a UDP-glucose dehydrogenase, preferably the UDP-glucose dehydrogenase encoded by the kfiD gene of *E. coli* K5.

5. The process as claimed in one of claims 1 to 4, **characterized in that** said cell further comprises a gene encoding an enzyme capable of synthesizing UDP-GalNAc (uridine-diphosphate-N-acetyl galactosamine), and the elements enabling the expression of said gene in said cell.

6. The process as claimed in one of claims 1 to 5, **characterized in that** said bacterium is a non-pathogenic *Escherichia coli* strain bacterium.

7. The process as claimed in one of claims 1 to 6, wherein said glycosaminoglycan is chondroitin.

8. The process for producing chondroitin in a genetically modified cell as claimed in claim 7, wherein the enzyme capable of synthesizing chondroitin from glucuronyl beta-galactoside is the bacterial chondroitin synthase from *Escherichia coli* strain K4 capable of synthesizing chondroitin from glucuronyl-lactose.

9. The process for producing chondroitin in a genetically modified cell as claimed in one of claims 7 or 8, said process comprising obtaining a bacterial cell of *Escherichia coli* strain, preferably a cell of *Escherichia coli* strain K12, said bacterial cell comprising at least:

   • an exogenous gene encoding a mammalian glucuronyl transferase, and the elements enabling the expression of said gene in said cell,
   • an exogenous gene encoding a bacterial chondroitin synthase, and the elements enabling the expression of said gene in said cell,
   • an exogenous gene encoding a lactose permease, and the elements enabling the expression of said gene in said cell,
   • an exogenous gene encoding a UDP-glucose dehydrogenase, and
   • an exogenous gene encoding UDP-N-acetylglucosamine epimerase from *Pseudomonas aeruginosa,*
   and the *wcaJ* (colanic acid synthesis enzyme) and *LacZ* ($\beta$-galactosidase) genes of said bacterial cell having been inactivated.

**10.** The process for producing chondroitin in a genetically modified cell as claimed in one of claims 7 to 9, said process comprising obtaining a bacterial cell of the *Escherichia coli* type, preferably a cell of the *Escherichia coli* strain K12, said cell comprising at least the *glcA-T* (beta-1-3-glucuronyl transferase), *kfoC* (chondroitin polymerase), *kfiD* (UDP-glucose 6-dehydrogenase) and *wbpP* (UDP-N-acetylglucosamine epimerase) genes.

**11.** The process as claimed in one of claims 1 to 6, **characterized in that** said glycosaminoglycan is heparosan.

**12.** The process for producing heparosan in a genetically modified cell as claimed in claim 11, said process comprising obtaining a bacterial cell of *Escherichia coli* strain, preferably a cell of *Escherichia coli* strain K12, said cell comprising at least: a) the *glcA-T* (beta-1-3-glucuronyl transferase) gene, and b) the *kfiA* (GlcNAc transferase), *kfiB, kfiC* (glcA transferase) and *kfiD* (UDP-glucose 6-dehydrogenase) genes from *E. coli* or at least one gene encoding a protein having a function homologous to that of a protein encoded by *kfiA, kfiB, kfiC* or *kfiD.*

**13.** Use of an *Escherichia coli* cell comprising at least the *glcA-T* (beta-1-3-glucuronyl transferase), *kfoC* (chondroitin polymerase), *kfiD* (UDP-glucose 6-dehydrogenase) and *wbpP* (UDP-N-acetylglucosamine epimerase) genes for the production of chondroitin.

**14.** Use of an *Escherichia coli* cell comprising at least the *glcA-T* (beta-1-3-glucuronyl transferase), *kfiA* (GlcNAc transferase), *kfiB, kfiC* (glcA transferase) and *kfiD* (UDP-glucose 6-dehydrogenase) genes for the production of heparosan.

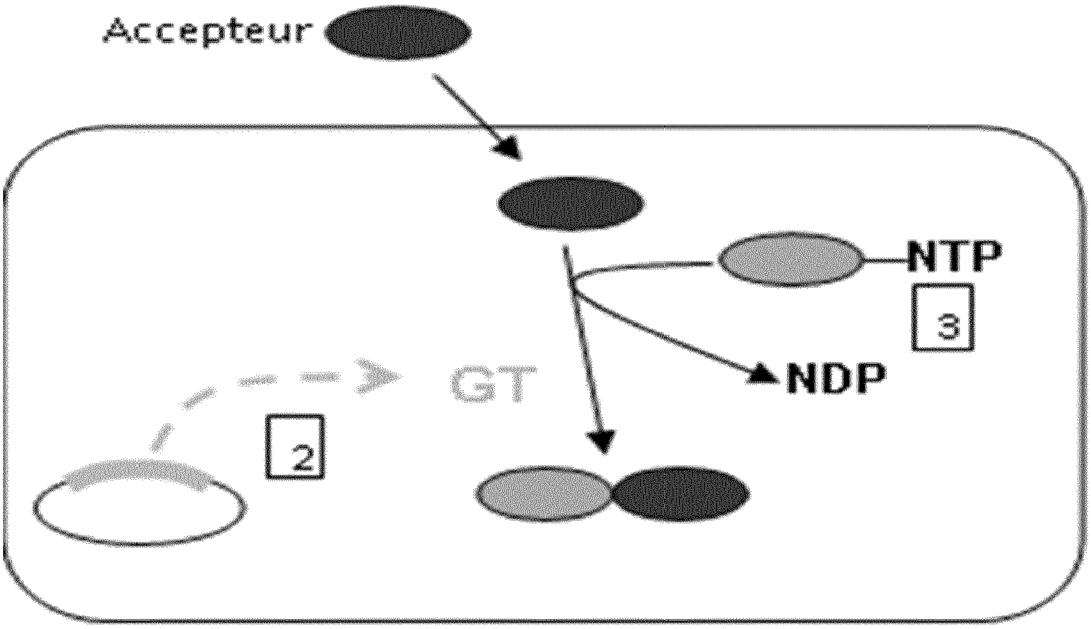


Figure 1

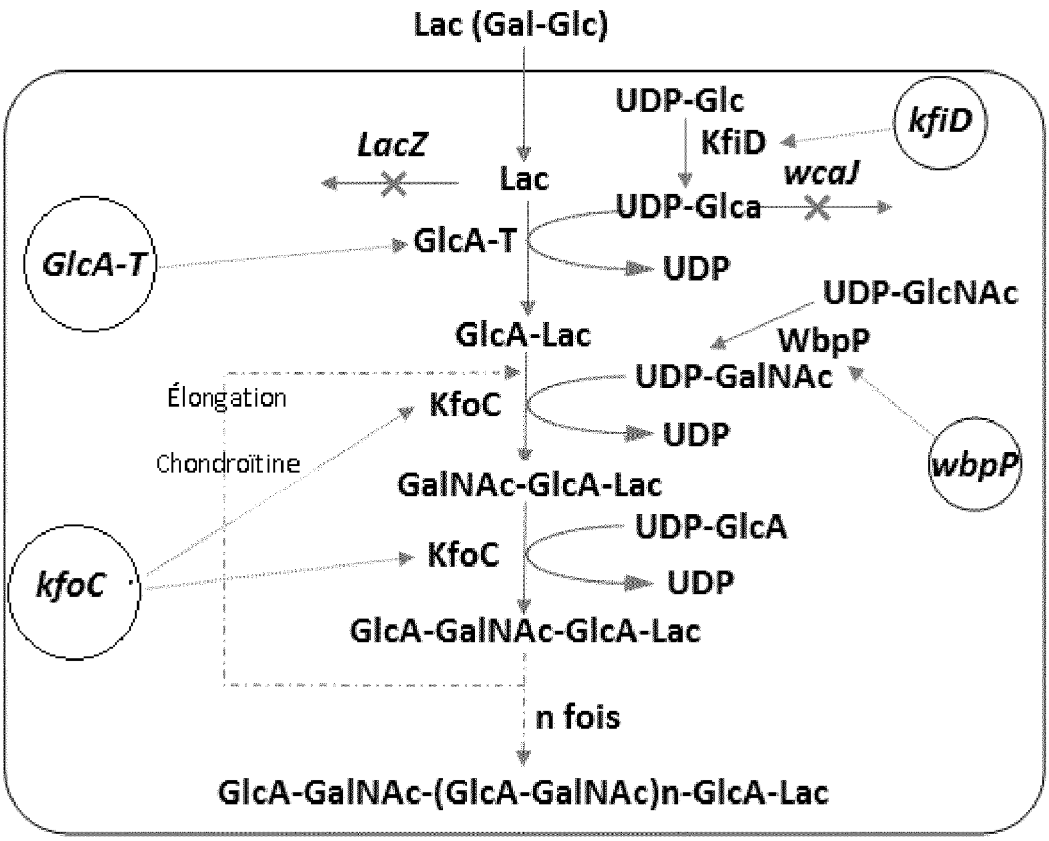


Figure 2

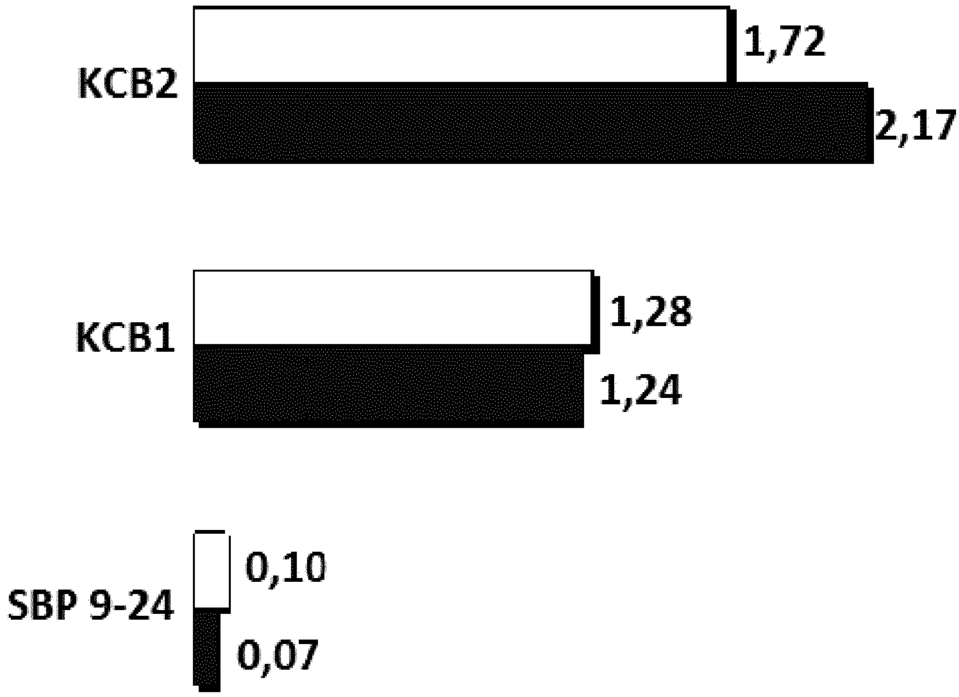


Figure 3

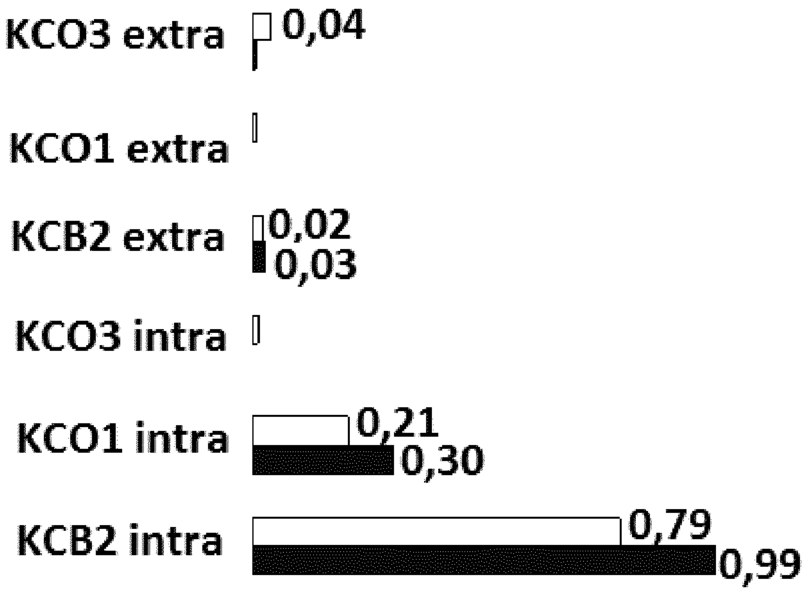


Figure 4

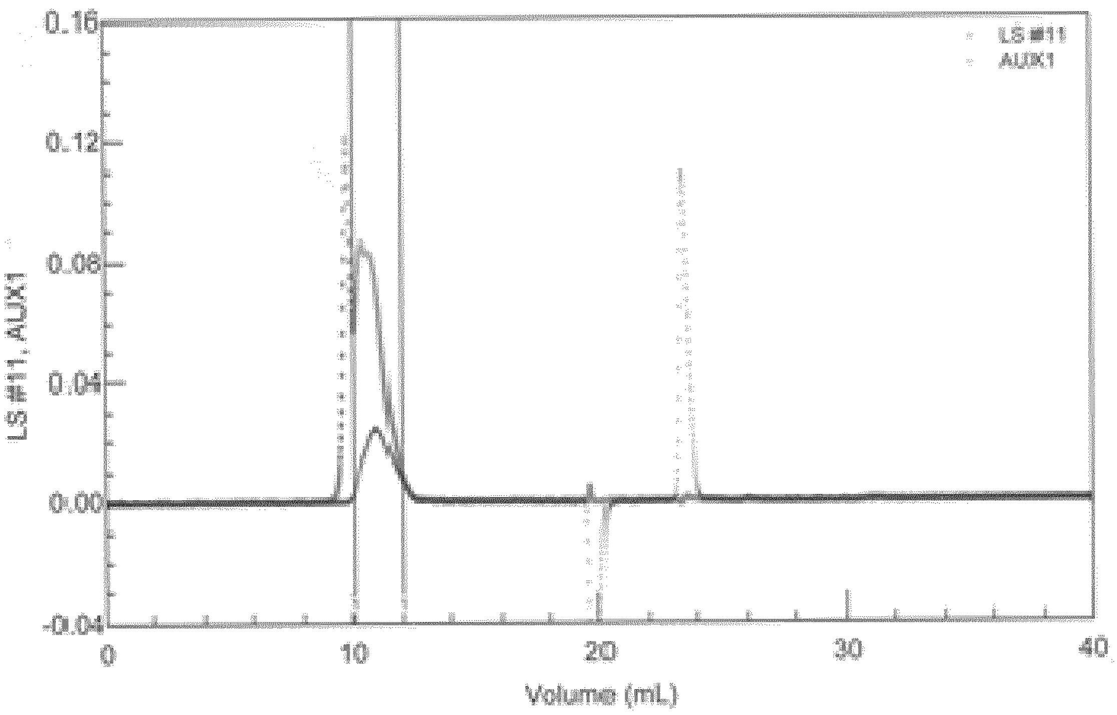


Figure 5A

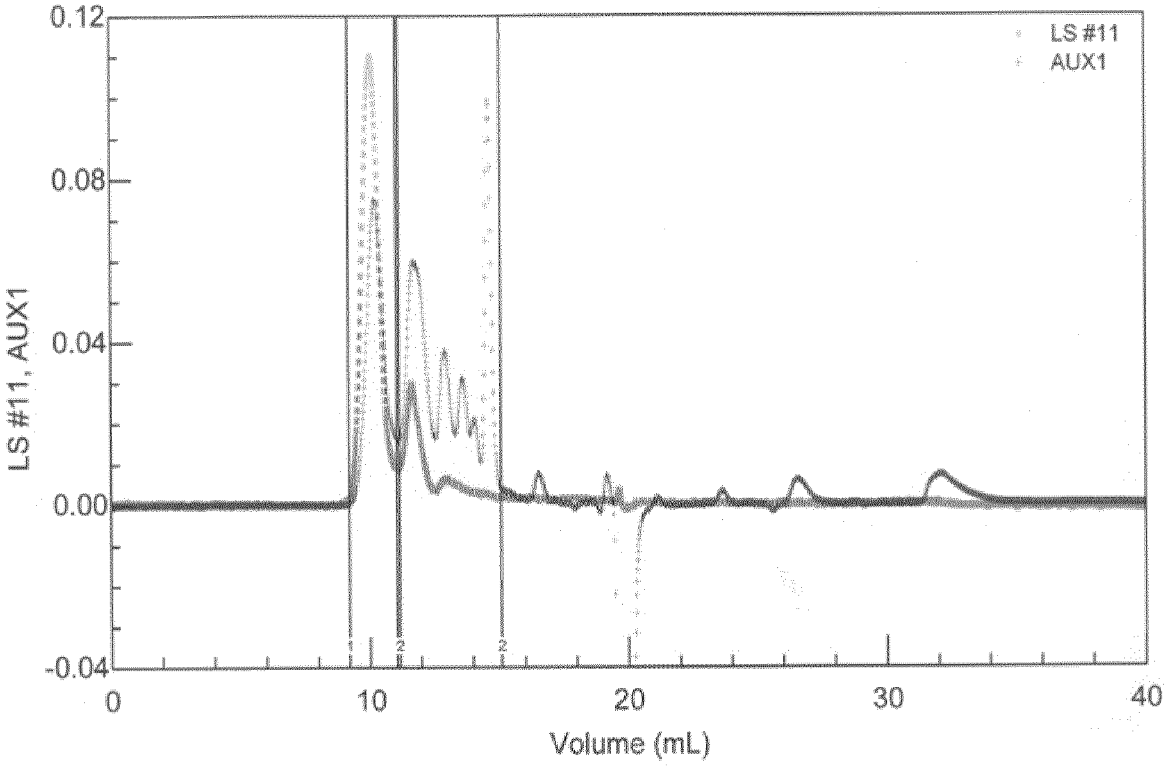


Figure 5B

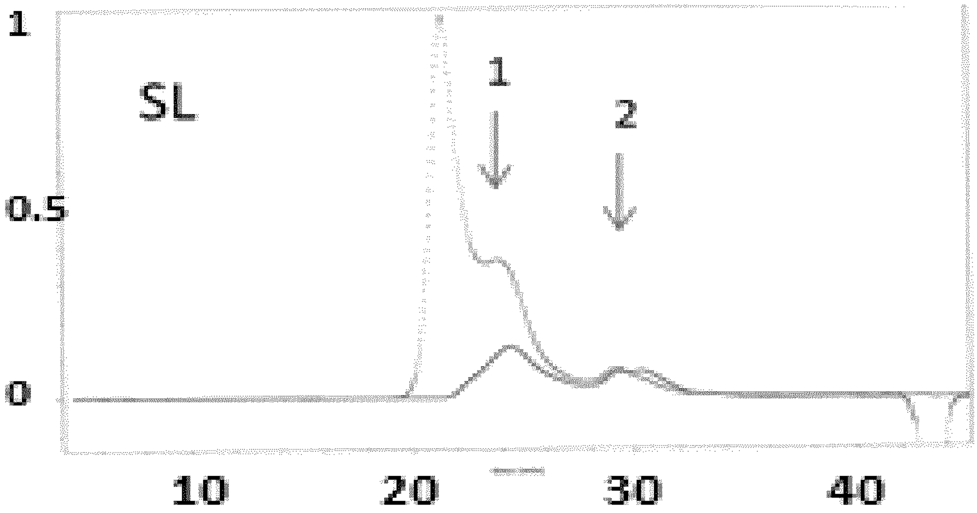


Figure 6A

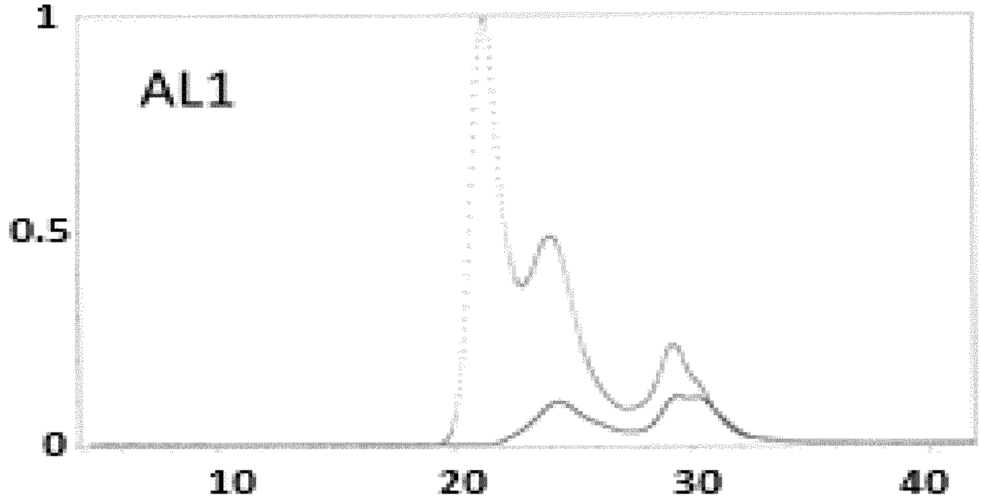


Figure 6B

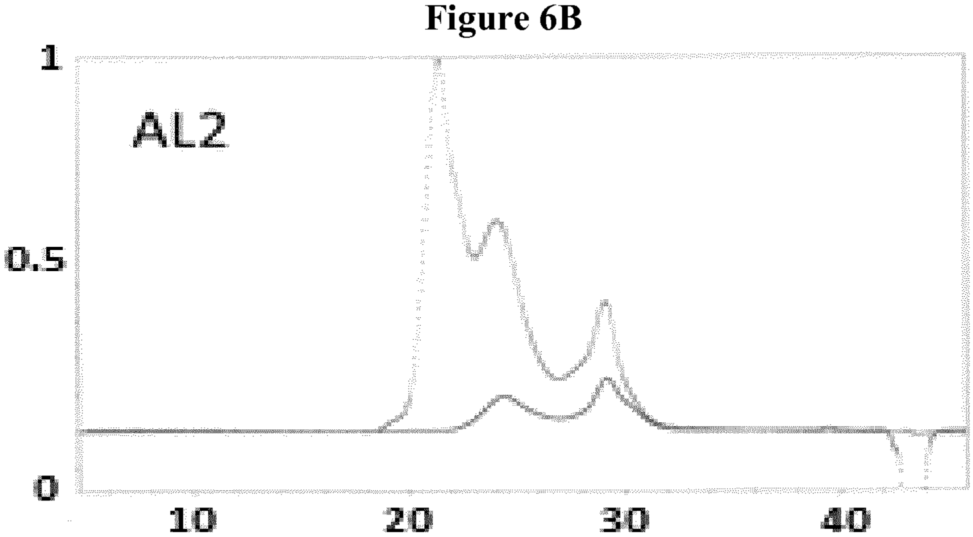


Figure 6C

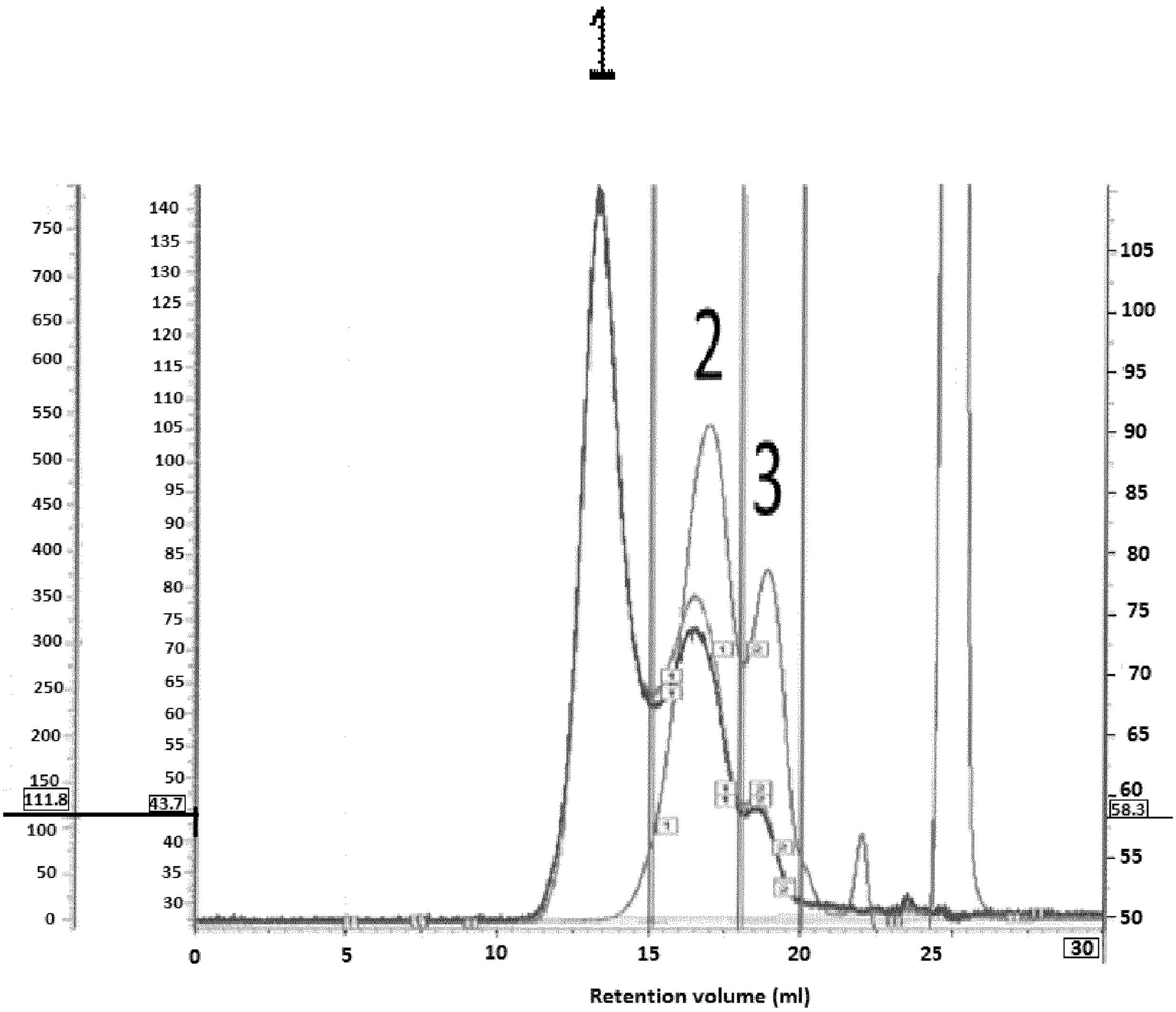
1
2
3
750
700
650
600
550
500
450
400
350
300
250
200
150
111.8
100
50
0
140
135
130
125
120
115
110
105
100
95
90
85
80
75
70
65
60
55
50
43.7
40
35
30
105
100
95
90
85
80
75
70
65
60
58.3
55
50
0
5
10
15
20
25
30
Retention volume (ml)

Figure 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0104341 A
- US 20110244520 A, E. Coli. Doherty
- WO 2012089777 A
- FR 1450564


### Littérature non-brevet citée dans la description


- **ROBINSON et al.** *The Biochemical Journal,* 01 Mai 1985, vol. 227 (3), 805-814
- **CIMINI et al.** *Appl. Microbiol, and Biotechnol,* 2010, vol. 85 (6), 1779-1787
- **JIN et al.** *Biosci. Biotechnol. Biochem.,* 2011, vol. 75 (7), 1283-1289
- **YAMADA ; SUGAHARA.** *Current Drug Discovery Technologies,* 2008, vol. 5, 289-301
- **ZANFARDINO et al.** *Microbiol. Cell Factories,* 2010, vol. 9, 34
- **WANG et al.** *Bioengineered Bugs,* Janvier 2011, vol. 2 (1), 63-67
- **SCHIRALDI et al.** *Appl. Microbiol, and Biotechnol,* 2010, vol. 87, 1209-1220
- **INOUÉ et al.** *Gene,* 1990, vol. 96, 23-28
- **YAVUZ et al.** *Glycobiology,* 2008, vol. 18 (2), 152-157
- **NINOMIYA et al.** *J. Biol. Chem.,* 14 Juin 2002, vol. 277 (24), 21567-75
- **PRIEM et al.** *Glycobiology,* 2002, vol. 12 (4), 235-240
- **KRÀHULEC et al.** *Molecular Biology,* 2005, vol. 30, 129-134
- **KAKUDA et al.** *J. Biol. Chem.,* 2004, vol. 279 (21), 22693-22703
- **WHITFIELD.** *Annual Review ofBiochemistry,* 2006, vol. 75, 39-68
- **MORGAN et al.** *Biochem. Journal,* 1955, vol. 61 (4), 586-589
- **BLUMENKRANTZ et al.** *Analytical biochemistry,* 1973, vol. 54, 484-489
- **MANZONI et al.** *J. Bioactive and Compatible Polymers,* 1996, vol. 11, 301
- **BARRETEAU et al.** *Carbohydrate Research,* 2012, vol. 1 (360), 19-24
- **HODSON et al.** *J. Biol. Chem.,* 2000, vol. 275 (35), 27311-5

SEQUENCE LISTING

<110> Centre National de la Recherche Scientifique (CNRS) Université Grenoble I Joseph Fourier

<120> Procédé de production in vivo de glycosaminoglycanne

<130> 366763 D32985

<150> FR 14 50564
<151> 2014-01-23

<160> 1

<170> PatentIn version 3.5

<210> 1
<211> 1692
<212> DNA
<213> Escherichia coli

<400> 1

atgatgaata aattagtgct agtcggacat cctggctcaa agtatcagat agttgaacat 60

tttttgaaag aaattggcat gaactcacca aattattcta caagtaataa aatttcccca 120

gaatatatca ccgcttcatt atgtcaattt tatcaaacac cagaagttaa tgatgtagta 180

gatgagagag aattctcagc tgttcaagtc tcaaccatgt gggatagcat ggttcttgaa 240

ctaatgatga acaatctaaa taacaaactt tgggggtggg cagatccatc tataatattt 300

tttcttgatt tttggaaaaa tatagataaa agcataaaat tcatcatgat atatgatcac 360

cctaaatata atttaatgcg ttcagtaaat aatgcccctc tctctttaaa tataaataat 420

agtgtagata actggattgc atataataaa agattgcttg attttttttt ggagaataaa 480

gaacgatgtg tgttgattaa ttttgaggcg tttcaaagca ataagaaaaa tattataaag 540

ccattgagta atattataaa aatagataat ctaatgtctg cgcattacaa aaattcaata 600

ttgtttgatg tggttgagaa taatgattat acaaaatcaa atgaaattgc cctgcttgaa 660

aaatatacaa ctttattttc tttaagtgca aatgagactg aaattacatt taatgataca 720

aaggttagtg agtacttagt atctgaatta ataaaagaaa gaaccgaggt tctgaagctt 780

tataatgagt tacaagccta tgcaaaccta ccttatatag aaacatcgaa agataacgtt 840

tcggctgagg ctgcattatg ggaggtagtc gaagagagaa attctatctt caatattgta 900

tctcatttgg tgcaagagtc aaaaaagaag gatgcagata ttgaattgac taaatctata 960

tttaagaaaa gacaattttt attattgaac aggattaatg agctaaaaaa agaaaaggaa 1020

gaggtaatta aactttcaaa aataaatcac aacgatgttg tgagacaaga aaaatatcca 1080

gatgatattg aaaaaaaaat aaatgacata cagaaatatg aagaagagat aagcgaaaaa 1140

gaatcaaaac tcactcaggc aatatcagaa aaagaacaga ttttaaaaca attgcataaa 1200

tatgaagaag agataagcga aaaagaatca aaactcactc aggcaatatc agaaaaagaa 1260

cagattttaa aacaattgca tatagtgcaa gagcagttgg aacactattt tatagaaaat 1320

caggaaatta aaaagaaact tccacctgtg ctatatggag cagctgagca gataaaacaa 1380

gagttaggtt atcgacttgg ttatattata gtctcgtatt ctaaatccct caaggggatt 1440

attaccatgc catttgcact tatccgtgag tgtgtttttg aaaaaaaacg taagaagagt 1500

tatggcgttg atgtgccact ctatttatat gctgatgctg ataaggctga aagagttaag 1560

aaacatttat cttatcaatt agggcaggct attatctcca gtgctaattc gatatttgga 1620

ttcattaccc ttccatttaa gttaattgtt gttgtttata aatataggag agctaaaatc 1680

aagggctgtt aa 1692